# EUROPEAN PATENT APPLICATION

(11) **EP 4 487 792 A1**
(43) Date of publication of application: **08.01.2025**
(21) Application number: 23770620.5
(22) Date of filing: 09.03.2023
(51) Int. Cl.: A61B 17/135

(54) **HEMOSTASIS TOOL**

(30) Priority: 18.03.2022 JP 2022044652
(71) Applicant: Terumo Kabushiki Kaisha, Tokyo 151-0072 (JP)
(72) Inventor: YAMASHITA, Koki, Fujinomiya-shi, Shizuoka 418-0015 (JP)
(74) Representative: Casalonga
(86) International application number: PCT/JP2023/008985
(87) International publication number: WO 2023/176664

(57) **Abstract**

There is provided a hemostatic device capable of preventing a proximal portion of a cover member from biting into a body surface of a patient wearing the hemostatic device, and capable of preventing an article or the like from being caught on the proximal portion of the cover member in a state where the hemostatic device is worn on the patient.

A main body 110 included in a hemostatic device 10 according to the present embodiment includes a retaining portion 130 that has a two-layer structure and retains a support member 300, the retaining portion includes an upper surface region 131 located on an upper surface 300b side of the support member, a lower surface region 132 that is located on a lower surface 300a side of the support member and faces the upper surface region across the support member, and a curved region 133 connecting the upper surface region and the lower surface region on a proximal side of the support member, the curved region is formed by folding a part of the main body toward the first band body 410 side, the lower surface region includes a constricted portion 132a on the side where the curved region is disposed, and a width of the lower surface region in the constricted portion decreases from the first band body side to the side where the curved region is disposed.

## Description

### Technical Field

The present invention relates to a hemostatic device.

### Background Art

As one of catheter procedures, there is known a procedure in which various medical elongated bodies are introduced into a blood vessel of a limb such as an arm or hand of a patient through a puncture site formed by puncturing the blood vessel to perform a diagnosis or a therapy at a lesion site. For example, Patent Literature 1 discloses a hemostatic device for stopping bleeding at a puncture site formed to enable access to a blood vessel (including a distal radial artery) running in a hand.

The hemostatic device of Patent Literature 1 includes a pressing member that applies a compressive force to a puncture site formed on a hand of a patient (hereinafter, also simply referred to as the "puncture site"), and a cover member configured to cover the pressing member. Furthermore, the cover member includes a plurality of band bodies for securing the pressing member to the hand of the patient, and a support member pressing the pressing member against the hand of the patient. The pressing member and the support member are disposed at predetermined positions of the cover member.

### Citation List

### Patent Literature

Patent Literature 1: US 2019/0133602 A

### Summary of Invention

### Technical Problem

When hemostasis is performed on the puncture site formed on the patient's limb, an operator such as a doctor (hereinafter, referred to as the "operator") disposes the hemostatic device on the patient's limb so as to cover the puncture site with the cover member. When the operator disposes the hemostatic device so as to cover the puncture site with the cover member, the pressing member and the support member located in the cover member are disposed at the puncture site. The hemostatic device can stop bleeding at the puncture site as the pressing member applies the compressive force to the puncture site in a state where the pressing member and the support member are disposed at the puncture site.

When hemostasis is performed using the above hemostatic device, the following problems may occur.

If the patient wearing the hemostatic device moves the limb during hemostasis performed by the pressing member, an edge portion of the cover member may bite into a body surface of the patient's limb. In particular, in a case where the limb on which the puncture site is formed is a hand, a proximal portion of the cover member (an end disposed on a forearm side) bites into a body surface near the wrist if the patient wearing the hemostatic device moves to twist the wrist toward a dorsal side of his or her hand or the like. Therefore, the hemostatic device may give the patient discomfort such as pain caused by the proximal portion of the cover member biting into the body surface of the limb.

Furthermore, in the hemostatic device, the pressing member is formed of an inflatable member that can be inflated by injection of a gas such as air. The inflatable member includes a part of the cover member and a sheet material connected to the cover member. Therefore, an edge portion of the sheet material forming the inflatable member is connected to the cover member. In such a case, the vicinity of the proximal portion of the cover member to which the sheet material is connected is harder than the other portion of the cover member along a width direction of the inflatable member. Therefore, the vicinity of the proximal portion of the cover member becomes harder along the width direction of the inflatable member in the hemostatic device, and an article or the like is easily caught on the proximal portion of the cover member in a state where the patient wears the hemostatic device. As the article or the like is caught on the proximal portion of the cover member in the hemostatic device, displacement of a mounting position of the hemostatic device is likely to occur. Furthermore, as the article is caught on the proximal portion of the cover member in the hemostatic device, discomfort may be given to the patient wearing the hemostatic device.

The present invention has been made based on the above problems, and an object thereof is to provide a hemostatic device capable of preventing a proximal portion of a cover member from biting into a body surface of a patient wearing the hemostatic device, and capable of preventing an article or the like from being caught on the proximal portion of the cover member in a state where the hemostatic device is worn on the patient.

### Solution to Problem

A hemostatic device according to the present invention includes: a cover member configured to cover a puncture site formed on a patient; a pressing member disposed on the cover member and configured to compress the puncture site; and a support member disposed on the cover member to cover the pressing member, wherein the cover member includes a main body in which the pressing member is located, a first band body which is configured to be disposed between fingers of the patient and extends in a first direction from the main body, a second band body extending in a second direction different from the first direction from the main body, and a third band body facing the second band body across the pressing member and extending in a third direction different from the first direction and the second direction from the main body, the main body includes a retaining portion that has a two-layer structure and retains the support member, the retaining portion includes an upper surface region located on an upper surface side of the support member, a lower surface region that is located on a lower surface side of the support member and faces the upper surface region across the support member, and a curved region connecting the upper surface region and the lower surface region on a proximal side of the support member, the curved region is formed by folding a part of the main body toward a side where the first band body is disposed, the lower surface region includes a constricted portion on a side where the curved region is disposed, and a width of the lower surface region in the constricted portion decreases from the side where the first band body is disposed to the side where curved region is disposed.

### Advantageous Effects of Invention

In the hemostatic device, the main body included in the cover member includes the retaining portion having the two-layer structure to retain the support member. The retaining portion has the curved region formed by folding a part of the main body toward the first band body side. In the hemostatic device, the curved region of the retaining portion comes into contact with a body surface when the patient moves a limb in a state where the hemostatic device is worn on the limb of the patient. Since the curved region is formed in a curved shape, the hemostatic device can prevent a proximal portion of the cover member from biting into the body surface of the patient. Furthermore, the hemostatic device has the lower surface region in which the retaining portion is disposed on the body surface side of the limb of the patient. The lower surface region includes the constricted portion in which the width of the lower surface region decreases toward the side where the curved region is disposed. In the state where the hemostatic device is worn on the limb of the patient, deformation of the upper surface region of the cover member is not inhibited by the lower surface region due to the constricted portion located in the lower surface region, and the upper surface region is deformed along the support member. Therefore, in the state where the hemostatic device is worn on the limb of the patient, the proximal portion of the cover member is deformed along both ends of the support member in a width direction in the hemostatic device, so that a width of the proximal portion of the cover member decreases. Since the width of the proximal portion of the cover member decreases, the hemostatic device can prevent an article or the like from being easily caught on the proximal portion of the cover member.

### Brief Description of Drawings

Fig. 1 is a view illustrating a hemostatic device according to a first embodiment, and is a plan view seen from an outer face side of each band body.
Fig. 2 is a view illustrating the hemostatic device according to the first embodiment, and is a plan view seen from an inner face side of each band body.
Fig. 3 is an enlarged view illustrating a part of the hemostatic device as seen from an outer surface side of a main body of a cover member.
Fig. 4A is an enlarged view illustrating a part of the hemostatic device as seen from an inner surface side of the main body of the cover member.
Fig. 4B is a view briefly illustrating a developed view of the cover member in a state before a retaining portion is formed.
Fig. 5 is an enlarged view illustrating a part of the hemostatic device as seen from the outer surface side of the main body of the cover member.
Fig. 6 is a partial cross-sectional view of the hemostatic device taken along an arrow 6A-6A illustrated in Fig. 5, and is a view illustrating a state when an inflatable portion inflates.
Fig. 7 is a partial cross-sectional view of the hemostatic device taken along an arrow 7A-7A illustrated in Fig. 5, and is a view illustrating a state when the inflatable portion inflates.
Fig. 8 is a plan view of a support member.
Fig. 9 is a perspective view of the support member as seen from a lower end side.
Fig. 10 is a perspective view of the support member as seen from an upper end side.
Fig. 11 is a perspective view illustrating a lower surface side of the support member.
Fig. 12 is a perspective view illustrating the lower surface side of the support member.
Fig. 13 is a view illustrating a hand (right hand) of a patient for which the hemostatic device is to be used.
Fig. 14 is a view briefly illustrating a use example of the hemostatic device.
Fig. 15 is a view briefly illustrating the use example of the hemostatic device.
Fig. 16 is a view briefly illustrating the use example of the hemostatic device.
Fig. 17 is a partial cross-sectional view taken along an arrow 17A-17A illustrated in Fig. 16.
Fig. 18 is a partial cross-sectional view taken along an arrow 18A-18A illustrated in Fig. 16.
Fig. 19 is a view illustrating the right hand of the patient wearing the hemostatic device.
Fig. 20 is an enlarged view illustrating a part of a hemostatic device according to a modification.
Fig. 21 is an enlarged view illustrating a part of a hemostatic device according to a second embodiment.
Fig. 22 is a partial cross-sectional view taken along an arrow 22A-22A illustrated in Fig. 21.

### Description of Embodiments

### <First Embodiment>

Hereinafter, a first embodiment of the present invention will be described with reference to the accompanying drawings. The following description does not limit the technical scope or the significance of each term disclosed in the claims. Furthermore, dimensional ratios in the drawings are exaggerated for convenience of description, and may be different from actual ratios.

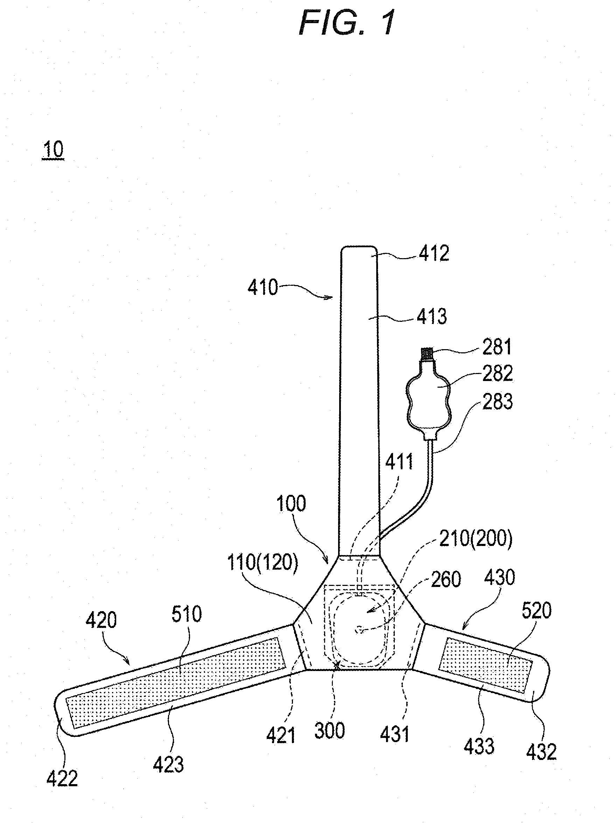

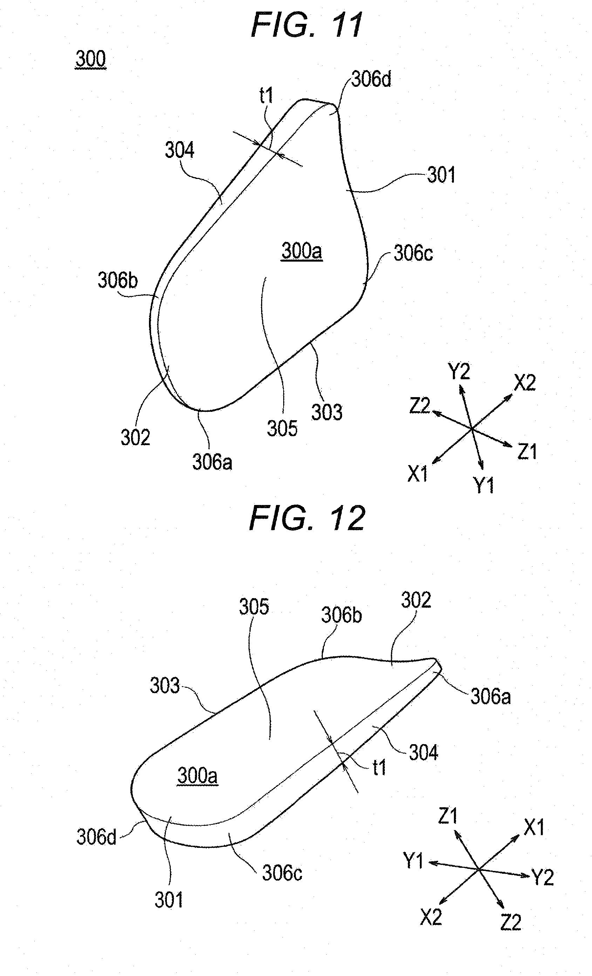

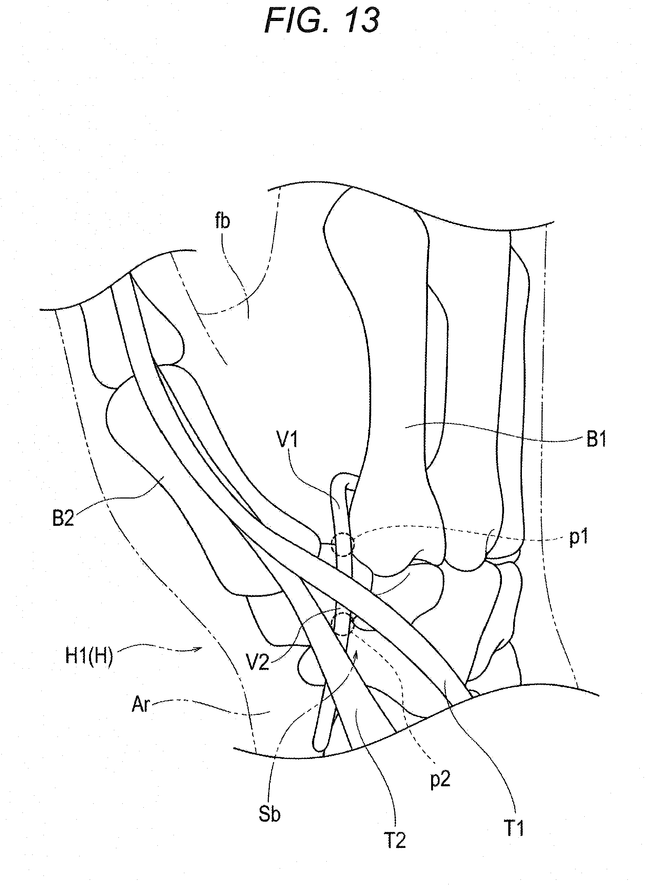

Figs. 1 to 12 are views for describing a hemostatic device 10 according to the first embodiment. Figs. 13 to 19 are views for describing a use example of the hemostatic device 10 according to the first embodiment.

For example, as illustrated in Figs. 13, 16, 17, 18, and 19, the hemostatic device 10 can be used to stop bleeding at a first puncture site p1 formed in a hand H located on a distal side (fingertip side) of a forearm Ar of a patient when a sheath tube 610 of an introducer 600 placed at the first puncture site p1 is removed.

A specific position of a puncture site where bleeding is to be stopped by the hemostatic device 10 is not particularly limited, but in the present specification, the first puncture site p1 and a second puncture site p2 are exemplified.

As illustrated in Figs. 13, 16, 17, 18, and 19, the first puncture site p1 is a puncture site formed in a distal radial artery (hereinafter, also referred to as a "blood vessel V1") located on a distal side of a snuff box Sb of a palmar artery running on a dorsal side of a right hand H1 located on the distal side of the forearm Ar of the patient (hereinafter, also simply referred to as the "right hand H1"). Furthermore, the first puncture site p1 is located at a predetermined position avoiding a metacarpal bone B1 of an index finger and a metacarpal bone B2 of a thumb between the metacarpal bone B1 of the index finger and the metacarpal bone B2 of the thumb. Note that the snuff box Sb is a cavity of the hand located near a radius when the patient spreads the thumb of the hand H.

As illustrated in Fig. 13, the second puncture site p2 is a puncture site formed in an artery V2 located in the snuff box Sb of the palmar artery running on the dorsal side of the right hand H1. The second puncture site p2 is located on the forearm Ar side (proximal side) of the patient with respect to the first puncture site p1, and is located between an extensor pollicis longus muscle T1 and an extensor pollicis brevis muscle T2 located on the dorsal side of the right hand H1.

Furthermore, the second puncture site p2 is located at a predetermined position avoiding a position of the metacarpal bone B1 of the index finger and a position of the metacarpal bone B2 of the thumb.

Note that the hemostatic device 10 can also be applied to hemostasis of a puncture site on a left hand of a patient formed at a position corresponding to the first puncture site p1 exemplified with the right hand H1 of the patient or a puncture site on the left hand of the patient formed at a position corresponding to the second puncture site p2 exemplified with the right hand H1.

Hereinafter, the hemostatic device 10 will be described in detail.

### <Hemostatic Device>

As schematically illustrated in Figs. 1, 2, 16, 17, 18, and 19, the hemostatic device 10 includes a cover member 100 configured to cover the first puncture site p1, a pressing member 200 disposed on the cover member 100 and configured to compress the first puncture site p1, and a support member 300 disposed on the cover member 100 so as to cover the pressing member 200.

In the present specification, the fingertip side of a finger is defined as a "distal side", and the forearm Ar side is defined as a "proximal side" in a state where the hemostatic device 10 is worn on the right hand H1. A "distal side" and a "proximal side" used in the description of the hemostatic device 10 are also defined similarly to directions defined above.

### <Pressing Member>

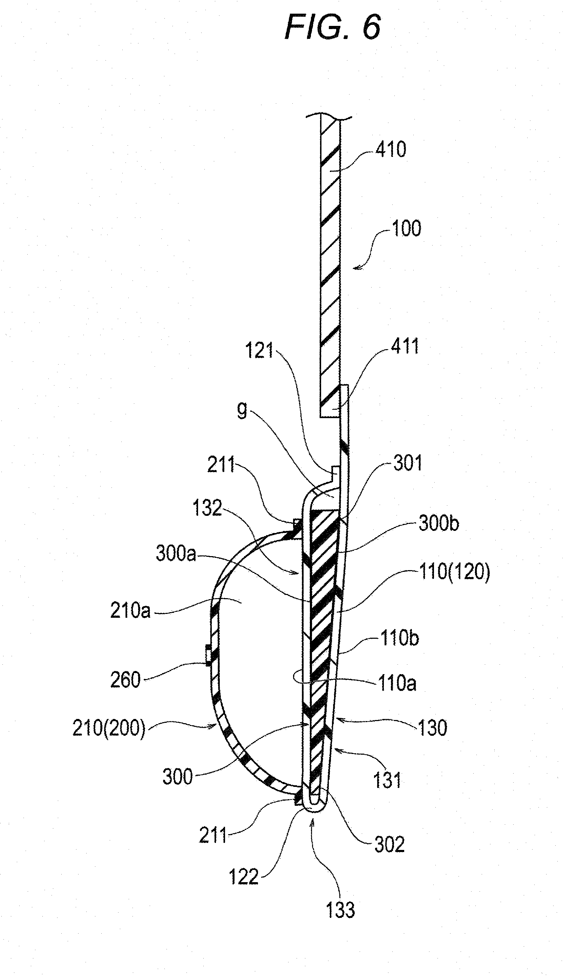

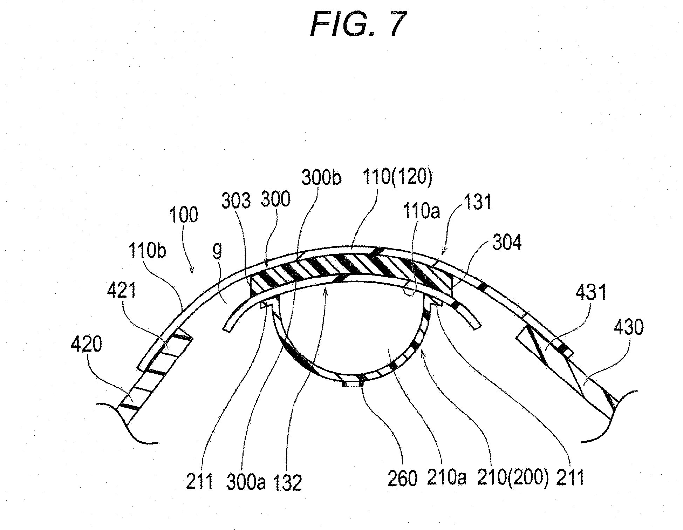

For example, as illustrated in Figs. 6 and 7, the pressing member 200 can be formed of an inflatable member 210 that can be inflated by injection of a fluid. In the present embodiment, an example in which the pressing member 200 is formed of the inflatable member 210 will be described in the following description.

The inflatable member 210 includes an inner cavity 210a into which the fluid can be injected. The inflatable member 210 is configured to inflate as the fluid is injected into the inner cavity 210a and deflate as the fluid injected into the inner cavity 210a is discharged. The fluid used for the inflation of the inflatable member 210 is, for example, a gas such as air. Figs. 6 and 7 illustrate cross-sectional views of the inflatable member 210 in an inflated state.

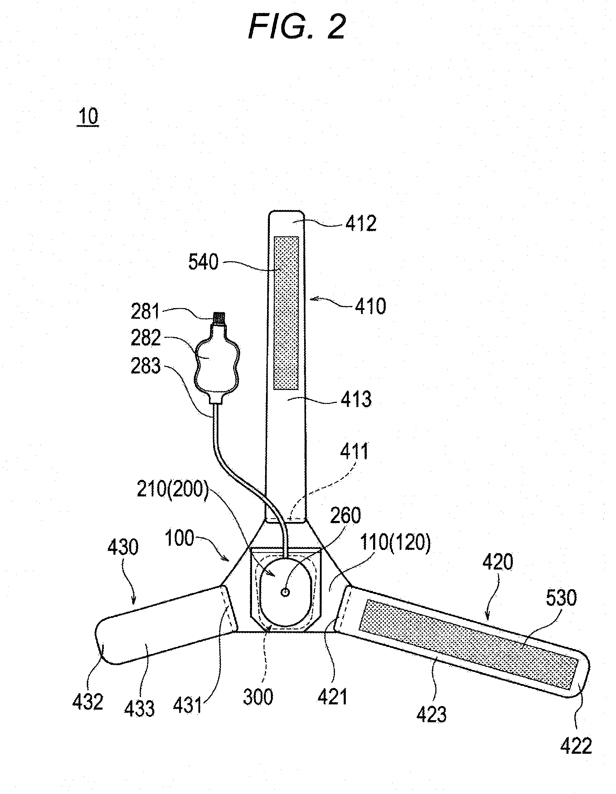

A tube 283 (see Figs. 1 and 2) to be described later is coupled to the inner cavity 210a of the inflatable member 210.

As illustrated in Figs. 6 and 7, the inflatable member 210 can be formed of, for example, a film-shaped member (sheet material) connected to a sheet material 120 forming a main body 110 of the cover member 100.

The inflatable member 210 can be disposed to form the inner cavity 210a against a lower surface region 132 of a retaining portion 130 formed by the sheet material 120.

An outer peripheral edge of the film-shaped member forming the inflatable member 210 forms an edge portion 211 of the inflatable member 210. The edge portion 211 of the inflatable member 210 can be connected to the sheet material 120 by, for example, fusing bonding, adhesion, or the like.

The film-shaped member forming the inflatable member 210 can be made of, for example, a resin material having a predetermined thickness.

A material of the film-shaped member forming the inflatable member 210 is not particularly limited, and for example, polyvinyl chloride, polyethylene, polypropylene, polybutadiene, polyolefins such as ethylene-vinyl acetate copolymer (EVA), polyesters such as polyethylene terephthalate (PET) and polybutylene terephthalate (PBT), various thermoplastic elastomers such as polyvinylidene chloride, silicone, polyurethane, polyamide elastomer, polyurethane elastomer, polyester elastomer, nylon, nylon elastomer, or any combination thereof (such as a blend resin, a polymer alloy and a laminate) can be used.

As illustrated in Figs. 6 and 7, the inflatable member 210 is disposed on an inner surface 110a side of the main body 110 of the cover member 100.

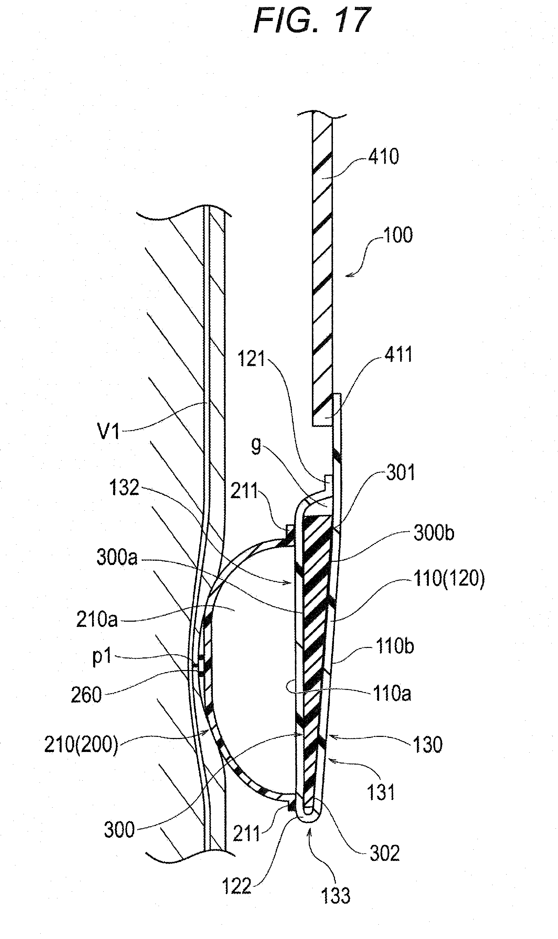

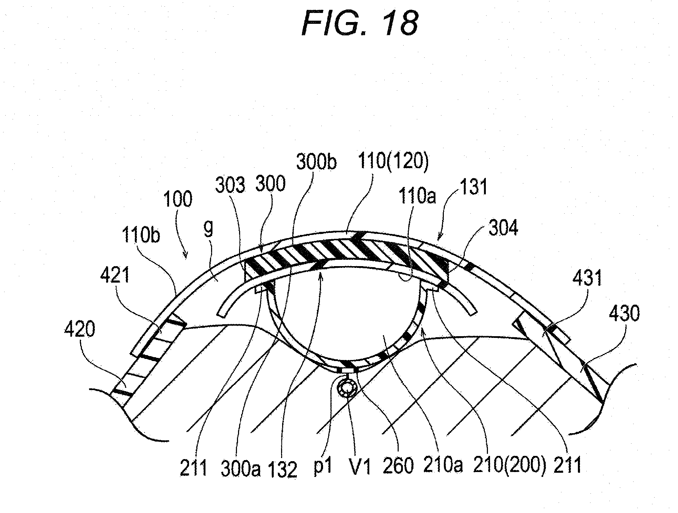

In the present embodiment, the inner surface 110a of the main body 110 is formed of a surface disposed on a body surface side of the right hand H1 in the lower surface region 132 of the retaining portion 130 (see Figs. 17 and 18). An outer surface 110b of the main body 110 is formed of a surface on a surface facing the outside in an upper surface region 131 of the retaining portion 130.

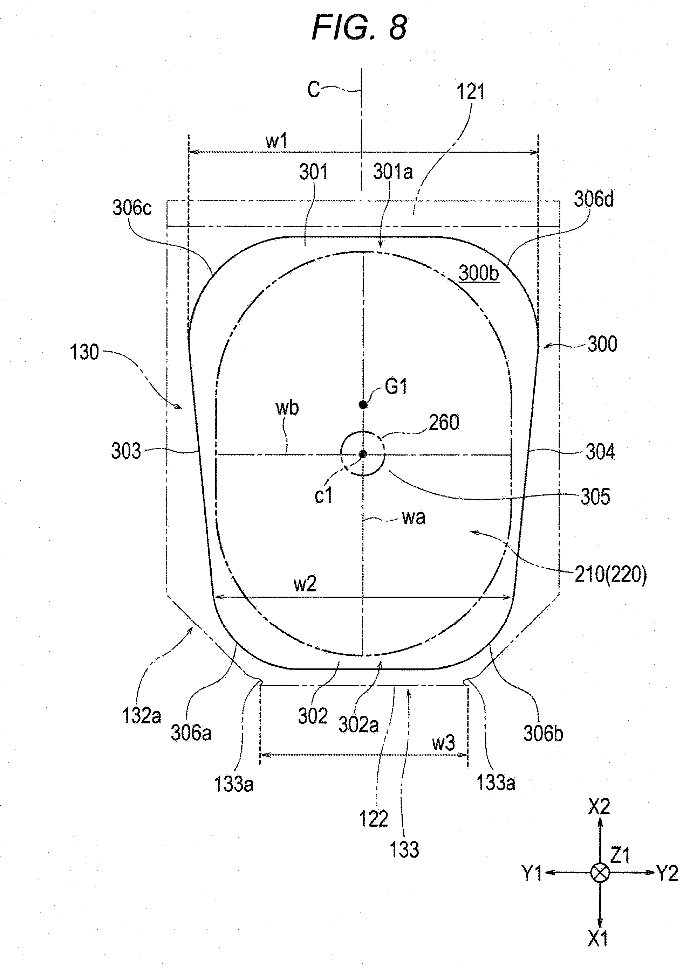

Fig. 8 illustrates a positional relationship among the inflatable member 210, the support member 300, and the retaining portion 130 in a deflated state on a plan view of the support member 300. The inflatable member 210 and the retaining portion 130 are indicated by two-dot chain lines. Note that Fig. 8 is a plan view of support member 300 as seen from an upper surface 300b side.

The inflatable member 210 has a longitudinal width wa passing through a center c1 of the inflatable member 210 and a lateral width wb that is orthogonal to the longitudinal width wa and passes through the center c1 of the inflatable member 210 in a state where the inflatable member 210 is deflated. Note that the longitudinal width wa and the lateral width wb are dimensions that do not include the edge portion 211 located on the outer periphery of the inflatable member 210, and mean the longitudinal width wa and the lateral width wb in an outer shape of the inflatable member 210 illustrated in the plan view illustrated in Fig. 8.

The center c1 of the inflatable member 210 is located at a center of an outer shape illustrated in the plan view of Fig. 8. In the present embodiment, the center c1 is located at a position where a center position of an axis (major axis) along the longitudinal width wa and a center position of an axis (minor axis) along the lateral width wb intersect. Note that, in the present embodiment, the center position of the axis (major axis) along the longitudinal width wa is a line segment passing through a point where distances from both ends of the axis along the longitudinal width wa are equal on the axis along the longitudinal width wa. Furthermore, the center position of the axis (minor axis) along the lateral width wb is a line segment passing through a point where distances from both ends of the axis along the lateral width wb are equal on the axis along the lateral width wb.

In the inflatable member 210, the lateral width wb is shorter than the longitudinal width wa. In the present embodiment, the inflatable member 210 has a substantially oval shape that is rotationally symmetric with respect to the center c1 in the plan view illustrated in Fig. 8.

In the plan view illustrated in Fig. 8, the inflatable member 210 is disposed on the cover member 100 in a state where the center c1 of the inflatable member 210 is located on the a lower end 302 side of the support member 300 with respect to a center of gravity G1 of an outer shape of the support member 300.

In the plan view illustrated in Fig. 8, the inflatable member 210 is connected to the lower surface region 132 such that a part (a part on a proximal portion side) of the inflatable member 210 is located in a constricted portion 132a of the retaining portion 130 (see Fig. 6).

Note that a planar shape of the inflatable member 210 is not particularly limited. For example, the inflatable member 210 may have a planar shape such as a circle, an ellipse, or a rectangle. When the inflatable member 210 is configured to have a planar shape such as a circular shape, an elliptical shape, or a rectangular shape, the center of the inflatable member 210 can be defined by a center position of the outer shape in the plan view.

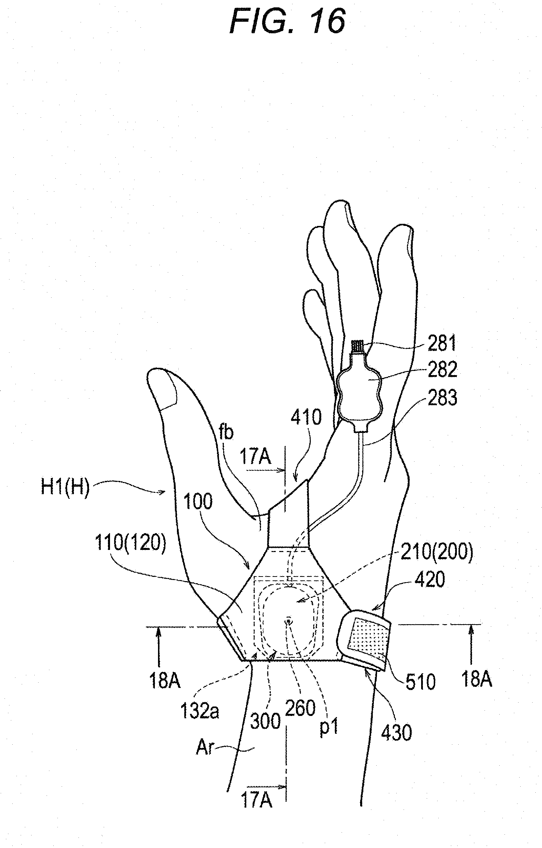

As illustrated in Figs. 16, 17, and 18, the inflatable member 210 includes a marker portion 260 for aligning the inflatable member 210 with the first puncture site p1.

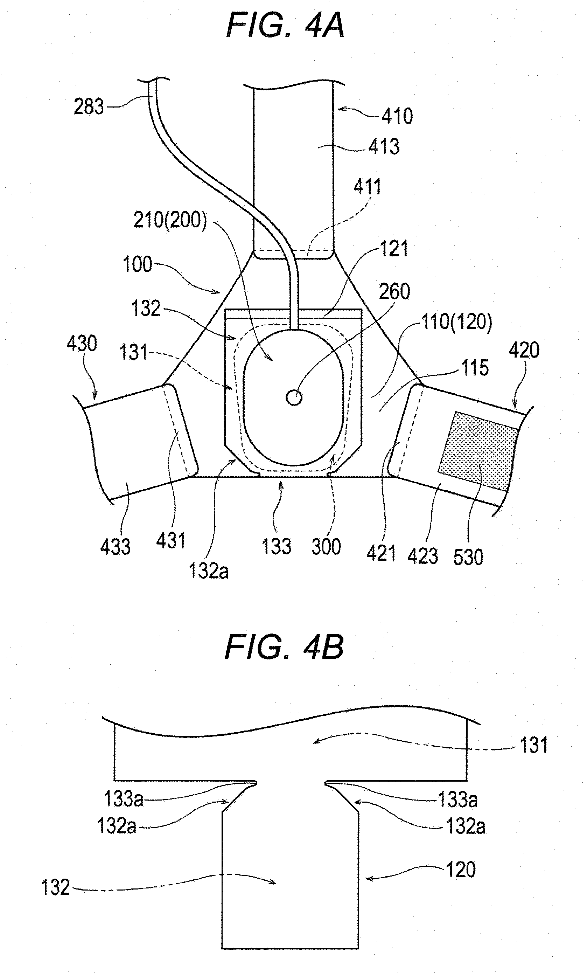

As illustrated in Figs. 4A and 8, the marker portion 260 can include a transparent central portion surrounding the center c1 and a colored circular frame portion surrounding the central portion.

As illustrated in Figs. 6 and 7, the marker portion 260 is disposed on an outer face of the inflatable member 210. Note that the marker portion 260 may be disposed on an inner face of the inflatable member 210 facing the inner cavity 210a.

Furthermore, specific shape and color, a position in a plane direction of the inflatable member 210, a formation method, and the like of the marker portion 260 are not particularly limited. The marker portion 260 can also include, for example, a circular marker configured entirely in color, a marker configured by a transparent central portion and a rectangular frame portion, a rectangular marker configured entirely in color, or the like.

### <Cover Member>

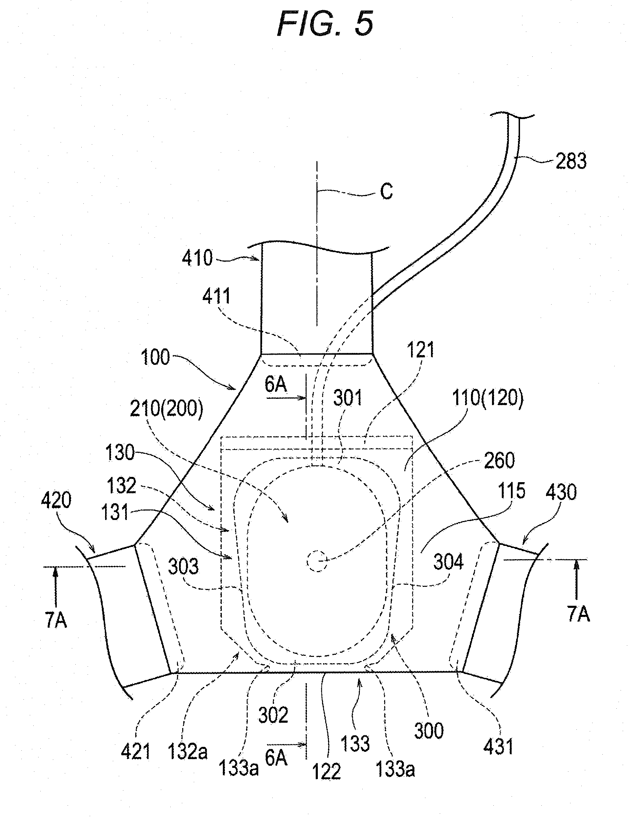

As illustrated in Figs. 5, 6, 7, 16, the cover member 100 includes the main body 110 in which the inflatable member 210 is located, a first band body 410 that is configured to be disposed between fingers of the patient and extends from the main body 110 in a first direction, a second band body 420 extending from the main body 110 in a second direction different from the first direction, and a third band body 430 facing the second band body 420 across the inflatable member 210 and extending from the main body 110 in a third direction different from the first direction and the second direction.

A region where the inflatable member 210 is disposed (a region overlapping the inflatable member 210 in a plan view illustrated in Fig. 5) in the cover member 100 forms the main body 110.

As illustrated in Figs. 6 and 7, the main body 110 has a two-layer structure and includes a retaining portion 130 retaining the support member 300.

The retaining portion 130 includes the upper surface region 131 located on the upper surface 300b side of the support member 300, the lower surface region 132 that is located on a lower surface 300a side of the support member 300 and faces the upper surface region 131 across the support member 300, and a curved region 133 connecting the upper surface region 131 and the lower surface region 132 on the proximal side of the support member 300 (the lower end 302 side of the support member 300).

The curved region 133 is formed by folding a part of the sheet material 120 forming the main body 110 toward the first band body 410 side. In the present embodiment, in the retaining portion 130, a part of the sheet material 120 is folded from the proximal side to the distal side such that an insertion portion g in which the support member 300 is disposed is formed in the main body 110 in the plan views illustrated in Figs. 5 and 8.

A first end 121 located on the distal side of a folded part of the sheet material 120 is connected to the sheet material 120 at a position on the distal side of an upper end 301 of the support member 300. The first end 121 forms a distal portion of the retaining portion 130.

Note that, in the sheet material 120, the vicinity of the first end 121 disposed on the upper end 301 side of the support member 300 is not necessarily linearly fusion-bonded along the first end 121. For example, the vicinity of the first end 121 can be fusion-bonded by a fused portion formed so as to surround the upper end 301 side of the support member 300 in a U shape. With such a configuration, the retaining portion 130 can stabilize a position of the support member 300 in the main body 110.

A proximal portion 122 of the cover member 100, formed of a part of the sheet material 120, is located in the curved region 133.

As illustrated in Fig. 6, in the retaining portion 130, the insertion portion g is closed between the first end 121 and the curved region 133 in a direction of the longitudinal width wa (see Fig. 8) of the inflatable member 210. Furthermore, as illustrated in Fig. 7, in the retaining portion 130, the insertion portion g communicates with the outside on the second band body 420 side and the third band body 430 side in a direction of the lateral width wb (see Fig. 8) of the inflatable member 210.

As illustrated in Figs. 4A, 5, and 8, the lower surface region 132 has the constricted portion 132a located on the curved region 133 side.

As illustrated in Figs. 4A and 8, in the constricted portion 132a, a width (lateral width) of the lower surface region 132 decreases from the first band body 410 side to the curved region 133 side. That is, the constricted portion 132a is configured such that the width of the lower surface region 132 gradually decreases from the distal side to the proximal side in the plan views illustrated in Figs. 4A and 8.

For example, as illustrated in Fig. 8, the constricted portion 132a can be configured such that the width decreases toward the curved region 133 side at substantially the same rate in a bilaterally symmetrical positional relationship with respect to a center line C of the first band body 410.

As illustrated in Fig. 8, a width w3 of the curved region 133 is smaller than a width w2 of the proximal portion (lower end 302) of the support member 300 in the plan view.

In the present specification, the width w2 of the proximal portion of the support member 300 can be defined by a length of a straight line connecting a boundary between the side surface portion 303 and the corner 306a and a boundary between the side surface portion 304 and the corner 306b. In a case where the corner 306a is formed in a curved shape as in the present embodiment, the boundary between the side surface portion 303 and the corner 306a is a position where a tangent line of the side surface portion 303 starts to change in a curved manner. Similarly, in a case where the corner 306b is formed in a curved shape as in the present embodiment, the boundary between the side surface portion 304 and the corner 306b is a position where a tangent line of the side surface portion 304 starts to change in a curved manner. Note that, in a case where the corners 306a and 306b are not formed in the curved shape, the width w2 of the proximal portion of the support member 300 can be defined by a length of a straight line connecting apexes located at the corners 306a and 306b.

As illustrated in Figs. 5 and 6, the retaining portion 130 is located on the proximal side of the main body 110.

The constricted portion 132a is located in an intermediate region 115 located between the second band body 420 and the third band body 430 in the main body 110. In the plan view illustrated in Fig. 5, the intermediate region 115 is a region where the second band body 420 and the third band body 430 overlap each other on the main body 110 when it is assumed that the second band body 420 is extended to the third band body 430 side or it is assumed that the third band body 430 is extended to the second band body 420 side.

As illustrated in Figs. 6 and 17, in the inflatable member 210, the edge portion 211 located on the proximal side of the inflatable member 210 is located in the curved region 133. That is, the edge portion 211 located on the proximal side of the inflatable member 210 is disposed so as to overlap the curved region 133 in the plan views illustrated in Figs. 5 and 8.

As illustrated in Figs. 5 and 8, the curved region 133 has curved portions 133a located at both ends in a width direction of the curved region 133. The curved portion 133a is curved in a shape projecting toward the center side in the width direction from each of both the ends of the curved region 133 in a plan view illustrated in Fig. 4B. Note that Fig. 4B illustrates a part of a developed view of the sheet material 120 in a state before the retaining portion 130 is formed.

As illustrated in Fig. 4A, each of the band bodies 410, 420, and 430 can be connected to any position in a region surrounding the periphery of the retaining portion 130 in the main body 110. Furthermore, each of the band bodies 410, 420, and 430 can be connected to a surface disposed on the body surface side of the right hand H1 in the above region.

Each of the band bodies 410, 420, and 430 can be connected to the main body 110 by, for example, fusion bonding or adhesion. Note that each of the band bodies 410, 420, and 430 may be integrally formed with the main body 110.

As illustrated in Figs. 1 and 2, the first band body 410 includes one end 411 connected to the main body 110 of the cover member 100, the other end 412 located opposite to the one end 411, and a main body 413 extending between the one end 411 and the other end 412.

For example, as illustrated in Fig. 16, the first band body 410 can be disposed so as to be hooked on an interdigital part fb located between the thumb and the index finger of the right hand H1 in a state where the inflatable member 210 is disposed at the first puncture site p1.

As illustrated in Figs. 1 and 2, the second band body 420 includes one end 421 connected to the main body 110 of the cover member 100, the other end 422 located opposite to the one end 421, and a main body 423 extending between the one end 421 and the other end 422.

The second direction in which the second band body 420 extends is not particularly limited as long as the direction is different from the first direction in which the first band body 410 extends.

As illustrated in Figs. 1 and 2, the third band body 430 includes one end 431 connected to the main body 110 of the cover member 100, the other end 432 located opposite to the one end 431, and a main body 433 extending between the one end 431 and the other end 432.

The third direction in which the third band body 430 extends is not particularly limited as long as the direction is different from the first direction in which the first band body 410 extends and the second direction in which the second band body 420 extends.

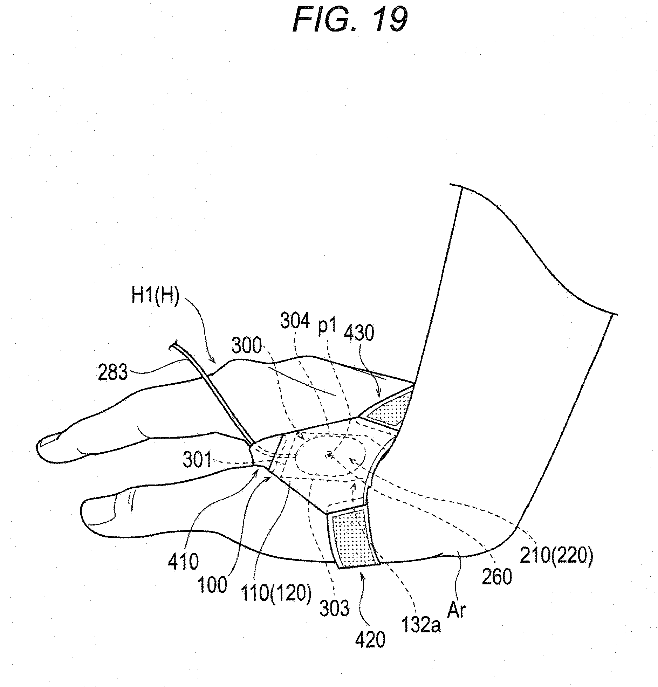

As illustrated in Figs. 18 and 19, the second band body 420 and the third band body 430 can be disposed so as to be wrapped along the outer periphery of the right hand H1 when the hemostatic device 10 is worn on the right hand H1.

As illustrated in Fig. 5, the second band body 420 and the third band body 430 are located at positions facing each other across the inflatable member 210. Therefore, when the second band body 420 and the third band body 430 are wrapped around the right hand H1, a tensile force in a direction of separating the second band body 420 and the third band body 430 from each other is applied to the both, and a tensile force in the same direction is also applied to the main body 110.

A constituent material of each of the band bodies 410, 420, and 430 is not particularly limited, and can be made of, for example, a vinyl chloride resin, a polyurethane resin, a polyester resin, or the like. Furthermore, a shape, a length, a thickness, and the like of each of the band bodies 410, 420, and 430 are not particularly limited.

Four securing parts, that is, a first securing part 510, a second securing part 520, a third securing part 530, and a fourth securing part 540, which enable the cover member 100 to be secured on the right hand H1, are disposed in the band bodies 410, 420, and 430, respectively.

As illustrated in Fig. 1, the first securing part 510 is disposed on an outer face of the main body 423 of the second band body 420.

As illustrated in Fig. 1, the second securing part 520 is disposed on an outer face of the main body 433 of the third band body 430.

As illustrated in Fig. 2, the third securing part 530 is disposed on an inner face of the main body 423 of the second band body 420.

As illustrated in Fig. 2, the fourth securing part 540 is disposed on an inner face of the main body 413 of the first band body 410.

The first securing part 510 and the second securing part 520 are formed of a male side of a hook-and-loop fastener. The third securing part 530 and the fourth securing part 540 are formed of a female side of the hook-and-loop fastener. The hook-and-loop fastener in the present specification is a hook-and-loop fastener, and is, for example, Magic Tape (registered trademark) or Velcro (registered trademark).

The second band body 420 and the third band body 430 are configured to be detachably attached via the third securing part 530 located on the inner face of the main body 423 of the second band body 420 and the second securing part 520 located on the outer face of the main body 433 of the third band body 430. Furthermore, the first band body 410 and the second band body 420 are configured to be detachably attached via the fourth securing part 540 located on the inner face of the main body 413 of the first band body 410 and the first securing part 510 located on the outer face of the main body 423 of the second band body 420.

Note that a specific structure of each of the securing parts 510, 520, 530, and 540 is not limited as long as the cover member 100 can be secured on the right hand H1 by connecting the band bodies 410, 420, and 430 to each other in a state where the hemostatic device 10 is disposed on the right hand H1. For example, omission of installation of some securing parts, a change of a position where the securing part is disposed in each of the band bodies 410, 420, and 430, and the like can be arbitrarily performed. Furthermore, in a case where each of the securing parts 510, 520, 530, and 540 is formed of the hook-and-loop fastener, the male side and the female side of the hook-and-loop fastener may be interchanged. Furthermore, the securing parts 510, 520, 530, and 540 may be configured using, for example, coupling mechanisms or the like including a frame portion in which a snap, a button, a clip, or a protrusion is formed and an engaged portion in which a hole engageable with the frame portion is formed.

### <Support Member>

Figs. 8 to 12 illustrate the support member 300. In the drawings, arrows X1 and X2 indicate a longitudinal direction of the support member 300 (the same direction as the direction of the longitudinal width wa of the inflatable member 210), arrows Y1 and Y2 indicate a width direction of the support member 300 (the same direction as the direction of the lateral width wb of the inflatable member 210), and arrows Z1 and Z2 indicate a thickness direction of the support member 300.

The support member 300 may be made of a material harder than the inflatable member 210. The support member 300 can be disposed to overlap the inflatable member 210 in the plan view illustrated in Fig. 5. The support member 300 is inserted into the insertion portion g located in the retaining portion 130.

As illustrated in Figs. 4A, 5, 8, 9, and 10, the support member 300 includes the upper end 301 that is located on the first band body 410 side and forms an end of the support member 300, the lower end 302 forming an end opposite to the upper end 301, a pair of side surface portions 303 and 304 connecting the upper end 301 and the lower end 302, and an intermediate portion 305 located between the upper end 301 and the lower end 302.

As illustrated in Figs. 16 and 19, the upper end 301 can be disposed on the fingertip side (distal side) of the finger in a state where the hemostatic device 10 is worn on the right hand H1. The lower end 302 can be disposed on the forearm Ar side (proximal side) in the state where the hemostatic device 10 is worn on the right hand H1. Note that the upper end 301 forms a distal portion of the support member 300, and the lower end 302 forms a proximal portion of the support member 300.

The upper end 301 of the support member 300 has a flat portion 301a that is linear in the plan view illustrated in Fig. 8. The flat portion 301a extends substantially linearly between corners 306c and 306d located on the upper end 301 side.

The lower end 302 of the support member 300 has a flat portion 302a that is linear in the plan view illustrated in Fig. 8. The flat portion 302a extends substantially linearly between corners 306a and 306b located on the lower end 302 side.

As illustrated in Fig. 8, the support member 300 is configured such that a width of the support member 300 decreases from the upper end 301 to the lower end 302. Therefore, the width w2 of the lower end 302 of the support member 300 is smaller than a width w1 of the upper end 301.

The support member 300 has a substantially trapezoidal shape in which the width w1 of the upper end 301 is larger than the width w2 of the lower end 302. The center of gravity G1 of the outer shape of the support member 300 is located at a position shifted from the intermediate portion 305 of the support member 300 to the upper end 301 side (a lower bottom side of the trapezoidal shape) by a predetermined distance.

In the present embodiment, the width w1 of the upper end 301 of the support member 300 is defined by a dimension (maximum width) of a part having the largest width in a region located on the upper end 301 side of the intermediate portion 305 of the support member 300.

As illustrated in Figs. 7, 9, and 10, the upper surface 300b of the support member 300 is curved toward the second band body 420 and the third band body 430. That is, the upper surface 300b of the support member 300 has a top portion protruding most near the center in the width direction of the support member 300. Furthermore, the top portion of the upper surface 300b of the support member 300 is located along the longitudinal direction of the support member 300. That is, the top portion of the upper surface 300b of the support member 300 is located along the longitudinal direction passing through the intermediate portion 305.

The upper surface 300b of the support member 300 can be formed to have a substantially constant radius of curvature at each portion in the longitudinal direction of the support member 300, for example.

As illustrated in Figs. 7, 11, and 12, the lower surface 300a of the support member 300 is curved in a shape projecting in a direction away from the inflatable member 210. Similarly to the upper surface 300b, the lower surface 300a is curved toward the second band body 420 and the third band body 430.

The lower surface 300a of the upper end 301 of the support member 300 can be formed to have a radius of curvature smaller than a radius of curvature of the lower surface 300a of the lower end 302 of the support member 300, for example.

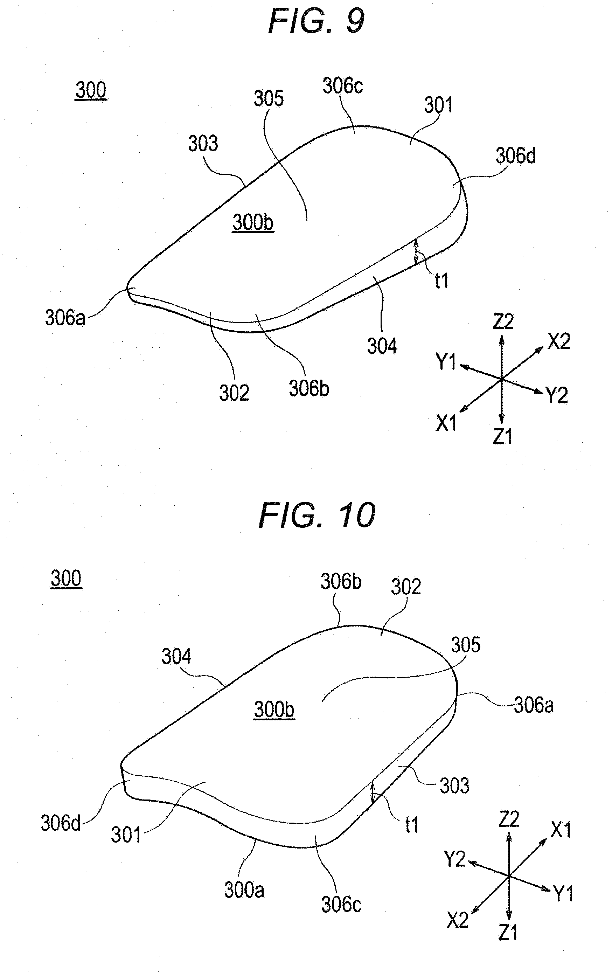

As illustrated in Figs. 10, 11, and 12, the support member 300 is configured such that a thickness t1 of each of the side surface portions 303 and 304 connecting the upper end 301 and the lower end 302 becomes thinner from the upper end 301 to the lower end 302. That is, the support member 300 is inclined such that a distance between the upper surface 300b and the lower surface 300a decreases from the upper end 301 to the lower end 302 in the cross-sectional view illustrated in Fig. 6 and a cross-sectional view illustrated in Fig. 17.

The support member 300 has a bilaterally symmetrical shape with respect to the center line C of the first band body 410 (an axis along the extending direction of the first band body 410) in the main body 110. In the present embodiment, the support member 300 has a bilaterally symmetrical shape with respect to a line segment that is orthogonal to an axis along the width w1 of the upper end 301 and passes through a point where distances from both ends of the width w1 of the upper end 301 are equal in the plan view of Fig. 8. Therefore, the support member 300 can be disposed on the main body 110 so as to be bilaterally symmetrical with respect to the center line C of the first band body 410 (the axis along the extending direction of the first band body 410). Furthermore, as illustrated in the plan view of Fig. 8, each of the side surface portions 303 and 304 is formed to be bilaterally symmetrical with respect to the center line C of the first band body 410 in the main body 110.

Each of the side surface portions 303 and 304 extends to be bilaterally symmetrical such that the width of the support member 300 decreases at a substantially constant rate from the upper end 301 side to the lower end 302 side.

Each of the corners 306a, 306b, 306c, and 306d located at the four corners of the support member 300 is formed in a curved shape that is rounded.

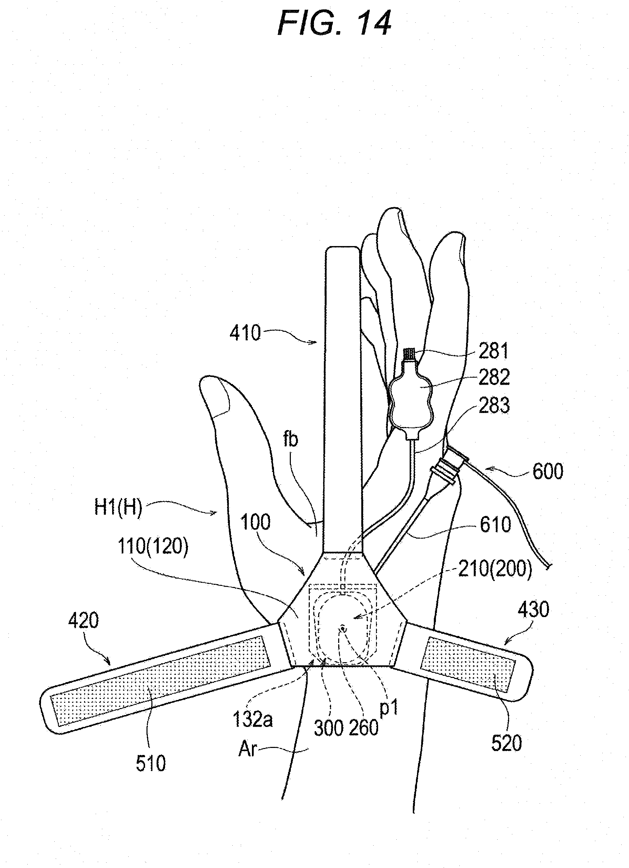

The hemostatic device 10 is preferably formed such that a part where the marker portion 260 of the inflatable member 210 is provided, a part overlapping the marker portion 260 in the cover member 100 (the upper surface region 131 and the lower surface region 132 of the retaining portion 130), and a part overlapping the marker portion 260 in the support member 300 are transparent. In a case where each of the members 100, 210, and 300 is configured in this manner, the operator can easily visually confirm a position of the marker portion 260 disposed on the inflatable member 210 and/or the first puncture site p1 via the parts formed to be transparent in the members 100, 210, and 300 when the hemostatic device 10 is worn on the right hand H1 as illustrated in Figs. 14 to 16. Note that the above term "transparent" includes colored transparent, colorless transparent, and translucent.

The support member 300 is made of a material harder than the inflatable member 210. Therefore, in the support member 300, when the inflatable member 210 applies a compressive force to the first puncture site p1 as illustrated in Figs. 17 and 18, the support member 300 can press the inflatable member 210 against the right hand H1. As a result, it is possible to prevent the inflatable member 210 from lifting-off from the right hand H1.

As a constituent material of the support member 300 when the inflatable member 210 is made of the above-described material, for example, acrylic resin, polyvinyl chloride (particularly hard polyvinyl chloride), polyolefin such as polyethylene, polypropylene, or polybutadiene, polystyrene, poly- (4-methylpentene-1), polycarbonate, ABS resin, polymethyl methacrylate (PMMA), polyacetal, polyacrylate, polyacrylonitrile, polyvinylidene fluoride, ionomer, acrylonitrile-butadiene-styrene copolymer, polyethylene terephthalate (PET), or the like, can be used.

### <Injection Portion>

As illustrated in Figs. 1 and 2, the hemostatic device 10 has an injection portion 281 for injecting a fluid into the inflatable member 210.

The injection portion 281 is formed of a connector incorporating a check valve (not illustrated). A syringe (not illustrated) can be connected to the injection portion 281.

A buffer member 282 having an inflatable space is disposed between the injection portion 281 and the inflatable member 210. The buffer member 282 is formed of a flexible bag-shaped member in which a space is formed. Note that the buffer member 282 may be provided with an arrow-shaped marker indicating an insertion direction of the syringe into the injection portion 281.

The injection portion 281 is connected to one end side of the buffer member 282. A lumen of the injection portion 281 communicates with the space of the buffer member 282. However, the communication between the lumen of the injection portion 281 and the space of the buffer member 282 is blocked while the check valve incorporated in the injection portion 281 is closed.

The tube 283 having flexibility is connected to the other end side of the buffer member 282. A lumen of the tube 283 communicates with the space of the buffer member 282. Furthermore, the other end of the tube 283, located opposite to one end connected to the buffer member 282, is connected to the inflatable member 210. The lumen of the tube 283 communicates with the inner cavity 210a of the inflatable member 210.

To inflate the inflatable member 210, the operator inserts a cylindrical distal end portion of the syringe (not illustrated) into the injection portion 281 and opens the check valve. The operator injects air in the syringe into the inner cavity 210a of the inflatable member 210 by pushing a pusher of the syringe in a state where the check valve of the injection portion 281 is opened.

When the air is injected into the inner cavity 210a of the inflatable member 210, the inflatable member 210 inflates. When the inflatable member 210 inflates, the buffer member 282 communicating with the inner cavity 210a of the inflatable member 210 via the tube 283 expands. The operator can easily grasp that the inflatable member 210 inflates without leakage of air by visually confirming the expansion of the buffer member 282.

To deflate the inflatable member 210, the operator inserts the cylindrical distal end portion of the syringe into the injection portion 281 and pulls the pusher of the syringe. The operator can discharge the air in the inner cavity 210a of the inflatable member 210 to the syringe by performing the above operation.

Note that the injection portion 281, the buffer member 282, and the tube 283 may be prepared and provided in a state of being coupled to the inflatable member 210, or may be prepared and provided in a state of being separated from the inflatable member 210. Furthermore, a specific configuration or the like of the injection portion 281 is not particularly limited as long as the supply of the fluid to the inner cavity 210a of the inflatable member 210 and the discharge of the fluid from the inner cavity 210a of the inflatable member 210 can be controlled.

### <Use Example of Hemostatic Device>

Next, a use example and operational effects of the hemostatic device 10 will be described with reference to Figs. 13 to 19.

Hereinafter, a process of using the hemostatic device 10 to stop bleeding at the first puncture site p1 formed on the right hand H1 illustrated in Fig. 13 will be described.

Fig. 14 illustrates a state where various procedures performed using the sheath tube 610 of the introducer 600 inserted into the first puncture site p1 are completed.

When causing the hemostatic device 10 to be worn on the right hand H1, the operator disposes the inflatable member 210 and the support member 300 so as to overlap the first puncture site p1 as illustrated in Fig. 14. At this time, the operator can appropriately align the inflatable member 210 and the support member 300 with the first puncture site p1 by disposing the marker portion 260 at the first puncture site p1 while visually confirming a position of the marker portion 260 disposed at the inflatable member 210.

When causing the hemostatic device 10 to be worn on the right hand H1, the operator can dispose the support member 300 so as to overlap the metacarpal bone B1 of the index finger and the metacarpal bone B2 of the thumb. An interval between the metacarpal bone B1 of the index finger and the metacarpal bone B2 of the thumb gradually narrows from the fingertip side to the forearm Ar side (see Fig. 13). A width of the support member 300 decreases from the upper end 301 disposed on the fingertip side to the lower end 302 disposed on the forearm Ar side (see Fig. 8). Therefore, the operator can dispose the support member 300 along the metacarpal bones B1 of the index finger and the metacarpal bones B2 of the thumb by disposing the support member 300 so as to overlap the metacarpal bones B1 of the index finger and the metacarpal bones B2 of the thumb from the dorsal side of the right hand H1. The operator can prevent a gap from being formed between the inflatable member 210 disposed on the lower surface 300a side of the support member 300 and the right hand H1 by disposing the support member 300 along the metacarpal bone B1 of the index finger and the metacarpal bone B2 of the thumb.

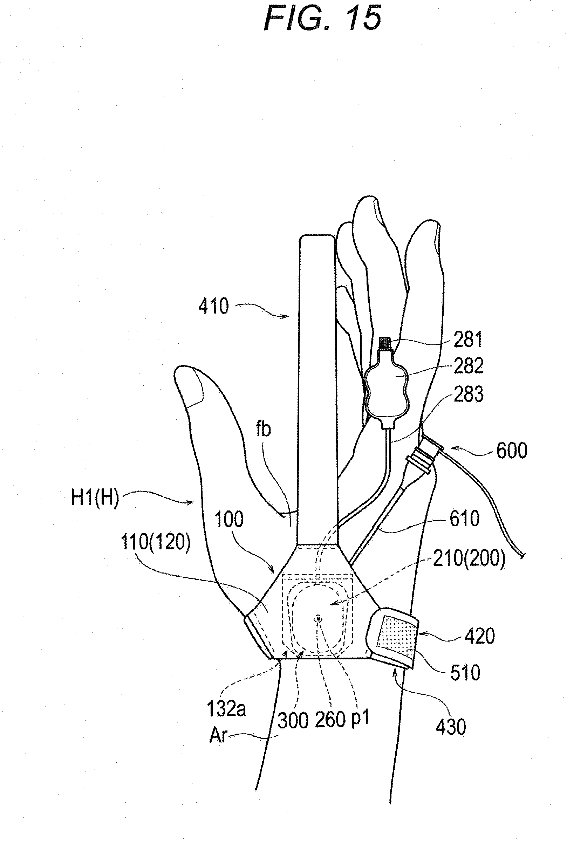

As illustrated in Fig. 15, the operator wraps the second band body 420 and the third band body 430 along the outer periphery of the right hand H1.

The operator can connect the second band body 420 and the third band body 430 via the securing parts 520 and 530 by bringing the third securing part 530 (see Fig. 2) disposed on a lower surface of the second band body 420 into contact with the second securing part 520 (see Fig. 1) disposed on an outer face of the third band body 430. The operator can firmly wrap each of the band bodies 420 and 430 along the outer periphery of the right hand H1 by connecting the band bodies 420 and 430 to each other in a state where the support member 300 overlaps the metacarpal bone B1 of the index finger and the metacarpal bone B2 of the thumb.

The upper surface 300b of the support member 300 is curved toward the second band body 420 side (the side surface portion 303 side) and the third band body 430 side (the side surface portion 304 side) (see Figs. 5, 7, 9, and 10). Therefore, the operator can press the support member 300 against the right hand H1 from the upper surface 300b side so as to be along the outer periphery of the right hand H1 as illustrated in Fig. 15 by wrapping the second band body 420 and the third band body 430 along the right hand H1 as illustrated in Fig. 18. Therefore, the hemostatic device 10 can enhance a force of securing the support member 300 and the inflatable member 210 with respect to the right hand H1.

As illustrated in Fig. 16, the operator disposes a part of the first band body 410 on the palm side of the right hand H1 while passing the first band body 410 through the interdigital part fb located between the thumb and the index finger of the right hand H1. At this time, the operator can connect the first band body 410 and the second band body 420 via the securing parts 510 and 540 by bringing the fourth securing part 540 (see Fig. 2) disposed on the inner face of the first band body 410 into contact with the first securing part 510 (see Fig. 1) disposed on the outer face of the second band body 420.

The support member 300 includes the upper end 301 and the side surface portions 303 and 304. The first band body 410 extends from the upper end 301 side, the second band body 420 extends from the side surface portion 303 side, and the third band body 430 extends from the side surface portion 304 side (see Fig. 5). Therefore, the hemostatic device 10 can secure the support member 300 to the right hand H1 in a state where a uniform force is applied to positions corresponding to the respective portions 301, 303, and 304 of the support member 300 by connecting the band bodies 410, 420, and 430 to each other.

The support member 300 is bilaterally symmetrical with respect to the center line C of the first band body 410 in the main body 110 (see Figs. 5 and 8). Therefore, the hemostatic device 10 can uniformly apply the force along the bilaterally symmetrical direction with respect to the support member 300 by wrapping each of the band bodies 420 and 430 extending to be bilaterally symmetrical with respect to the first band body 410 around the right hand H1. As a result, the hemostatic device 10 can prevent the support member 300 from being displaced in a state where the support member 300 is disposed on the right hand H1.

An inclined part exists in each portion (for example, the vicinity of the snuff box Sb) of the right hand H1. Furthermore, a shape of the inclined part of the right hand H1 depends on an individual difference of the patient. For example, in a case where the lower surface 300a of the support member 300 is formed in a flat shape, it is difficult to dispose the support member 300 along the inclined part of the right hand H1. In the hemostatic device 10, the lower surface 300a of the support member 300 is curved in the shape projecting in the direction away from the inflatable member 210 (see Figs. 7 and 18). Therefore, when the lower surface 300a of the support member 300 is disposed so as to overlap the inclined part of the right hand H1, the lower surface 300a of the support member 300 can be disposed along the right hand H1. As a result, the hemostatic device 10 can dispose the support member 300 to overlap the first puncture site p1 without depending on a dominant hand of the patient or the way of placement by the operator. Therefore, in the hemostatic device 10, the support member 300 can be suitably disposed at a desired position near the first puncture site p1.

Further, since the lower surface 300a of the support member 300 is disposed along the body surface of the right hand H1 in the hemostatic device 10 when the operator wraps the second band body 420 and the third band body 430 along the right hand H1, the band bodies 420 and 430 extending from the side surface portions 303 and 304 of the support member 300 can be disposed close to or in close contact with the body surface of the right hand H1 (see Fig. 18).

Therefore, the hemostatic device 10 can enhance a force of securing the support member 300 and the inflatable member 210 with respect to the right hand H1. As a result, even when an impact or the like is applied from the outside in the state where the hemostatic device 10 is worn on the right hand H1, the support member 300 and the inflatable member 210 are less likely to be displaced.

The operator inflates the inflatable member 210 by injecting air into the inflatable member 210 in a state where the syringe is connected to the injection portion 281. As illustrated in Figs. 17 and 18, when the inflatable member 210 inflates in the hemostatic device 10, the inflatable member 210 applies a compressive force to the first puncture site p1.

When the inflatable member 210 inflates in the state where the hemostatic device 10 is worn on the right hand H1, the support member 300 presses the inflatable member 210 against the right hand H1. As a result, the hemostatic device 10 can prevent the inflatable member 210 from lifting-off from the right hand H1.

When performing hemostasis using the hemostatic device, the operator can strongly tighten the first band body disposed in the interdigital part fb, for example, in order to enhance the force of securing the support member with respect to the right hand H1. The operator can firmly secure the support member to the right hand H1 by strongly tightening the first band body even if the support member is disposed in an unstable state on the right hand H1. However, the patient sometimes feels pain when the first band body is strongly tightened. Therefore, in a case where the first band body cannot be strongly tightened due to the pain felt by the patient or the like, the lower surface of the support member may be disposed to be inclined with respect to the body surface of the right hand H1. Specifically, the upper end of the support member on which the first band body is disposed lifts-off to a position excessively away from the body surface of the right hand H1 as compared with the lower end located opposite to the upper end. As a result, in the hemostatic device, the support member is disposed to be inclined such that a distance between the lower surface of the support member and the body surface of the right hand H1 gradually decreases from the upper end to the lower end of the support member.

The hemostatic device 10 is configured such that the thickness t1 of each of the side surface portions 303 and 304 of the support member 300 becomes thinner from the upper end 301 to the lower end 302. In addition, the first band body 410 configured to be disposed at the interdigital part fb located between the fingers of the patient extends from the upper end 301 side of the support member 300, and is configured to press the upper end 301 side of the support member 300 against the right hand H1. In the hemostatic device 10 configured as described above, when the inflatable member 210 starts to inflate in a state where the support member 300 and the inflatable member 210 are secured to the right hand H1, the inflatable member 210 applies a force to the vicinity of the upper end 301 of the support member 300. The upper end 301 side of the support member 300 is lifted by the force applied by the inflatable member 210. As a result, the support member 300 is disposed such that the lower surface 300a of the support member 300 is substantially parallel with the body surface of the right hand H1 in a state where the inflatable member 210 inflates as illustrated in Fig. 17. Therefore, the hemostatic device 10 can apply a compressive force in a direction perpendicular to the first puncture site p1 when the inflatable member 210 inflates.

The width w2 of the lower end 302 of the support member 300 is smaller than the width w1 of the upper end 301 of the support member 300 (see Fig. 8). Therefore, when the support member 300 is disposed on the right hand H1, the support member 300 can be disposed along the metacarpal bone B1 of the index finger and the metacarpal bone B2 of the thumb. Therefore, the hemostatic device 10 can firmly wrap the second band body 420 and the third band body 430 along the outer periphery of the right hand H1. Further, in the hemostatic device 10, the lower surface 300a of the support member 300 is curved in the shape projecting in the direction away from the inflatable member 210. Therefore, the inflatable member 210 is curved so as to fit along the lower surface 300a of the support member 300 in the state where the hemostatic device 10 is worn on the right hand H1. Thus, the hemostatic device 10 can effectively increase the compressive force applied to the first puncture site p1 by the inflatable member 210.

The retaining portion 130 is located on the proximal side of the main body 110 (see Fig. 5). The inflatable member 210 is connected to the lower surface region 132 such that a part of the inflatable member 210 is located at the constricted portion 132a (see Figs. 5 and 6). The inflatable member 210 connected to the retaining portion 130 is located on the proximal side of the main body 110 together with the retaining portion 130. Therefore, the center c1 of the inflatable member 210 is located at a position shifted to the proximal side of the main body 110 with respect to the center of gravity G1 of the support member 300 in the plan views illustrated in Figs. 5 and 8. For example, when the center c1 of the inflatable member 210 is disposed on the distal side of the center of gravity G1 of the support member 300 in the hemostatic device 10, the inflatable member 210 is likely to lift-off on a distal portion side of the main body 110 when the second band body 420 and the third band body 430 are wrapped along the outer periphery of the right hand H1 in order to cause the hemostatic device 10 to be worn on the right hand H1. When the inflatable member 210 lifts-off as described above in the hemostatic device 10, the compressive force applied to the first puncture site p1 by the inflatable member 210 decreases. According to the hemostatic device 10, it is possible to prevent the occurrence of such a decrease in the compressive force.

In the inflatable member 210, the edge portion 211 of the inflatable member 210 is located in the curved region 133 (see Fig. 6). Therefore, the center c1 of the inflatable member 210 is located at a position shifted to the curved region 133 side located on the proximal side of the main body 110. As a result, since the center c1 of the inflatable member 210 is disposed on the proximal side of the main body 110 with respect to the center of gravity G1 of the support member 300 in the hemostatic device 10, it is possible to more reliably prevent the inflatable member 210 from lifting-off on the distal portion side of the main body 110 when the second band body 420 and the third band body 430 are wrapped along the outer periphery of the right hand H1.

Note that, in the hemostatic device 10 according to the present embodiment, the curved region 133 is formed by bending the sheet material 120 forming the main body 110 at a position of the proximal portion 122 of the cover member 100. For example, it is also possible to configure a hemostatic device by connecting an inflatable member, formed by pasting two sheet materials to each other, to a sheet material having a flat shape in which the curved region 133 is not formed. However, when the inflatable member formed of the two sheet materials is connected to the sheet material having the flat shape, the inflatable member is connected to the sheet material having the flat shape with edge portions on the distal side and the proximal side of the sheet material on the outer face side forming the inflatable member as fusion margins. With such a configuration, a position where the inflatable member is disposed needs to be shifted to the distal side of the main body 110 by the amount of securing the proximal side of the sheet material forming the inflatable member as the fusion margin. As a result, the center c1 of the inflatable member 210 is shifted to the distal portion side of the main body 110 so as to approach the center of gravity G1 of the support member 300, there is a possibility that the main body 110 cannot be effectively prevented from lifting-off when the first band body 410 and the second band body 420 are wrapped around the right hand H1.

The curved region 133 has the curved portions 133a located at both the ends in the width direction of the curved region 133 (see Figs. 5 and 8). When the patient moves to twist the wrist or the like in the state where the hemostatic device 10 is worn on the right hand H1, both the ends in the width direction of the curved region 133 are deformed to be bent in the hemostatic device 10. The curved portion 133a of the curved region 133 alleviates concentration of a load on the constricted portion 132a when the load is applied to both the ends in the width direction of the curved region 133. Therefore, it is possible to prevent the curved region 133 from being damaged near the constricted portion 132a when both the ends in the width direction of the curved region 133 are deformed to be bent as described above.

The lower surface 300a of the support member 300 is curved in the shape projecting in the direction away from the inflatable member 210 (see Figs. 7 and 18). As illustrated in Fig. 18, the hemostatic device 10 is disposed such that the support member 300 is along the body surface of the right hand H1 in a state where the inflatable member 210 inflates. When the inflatable member 210 inflates, the hemostatic device 10 presses the inflatable member 210 along the body surface of the right hand H1 by the lower surface 300a of the support member 300. As a result, in the hemostatic device 10, the inflatable member 210 applies a compressive force in a direction toward the first puncture site p1 from a center side in the width direction of the lower surface 300a of the support member 300 and the side surface portions 303 and 304 located at both ends in the width direction of the lower surface 300a of the support member 300.

Therefore, the hemostatic device 10 can effectively enhance the compressive force applied to the first puncture site p1 by the inflatable member 210 as compared with a case where the lower surface 300a of the support member 300 is formed in a flat surface shape.

Fig. 19 illustrates a state where the patient twists the wrist to the dorsal side of the right hand H1 in a state where the hemostatic device 10 is worn on the right hand H1 and the inflated inflatable member 210 applies a compressive force to the first puncture site p1. In the state where the hemostatic device 10 is worn on the right hand H1, the curved region 133 having the constricted portion 132a is disposed on the forearm Ar side. When the patient moves to twist the wrist to the dorsal side of the right hand H1, the curved region 133 folded from the proximal side to the distal side of the main body 110 comes into contact with the body surface. The hemostatic device 10 can prevent the proximal portion 122 of the cover member 100 from biting into the body surface such as the wrist or the forearm Ar of the patient since the curved region 133 formed in a curved shape comes into contact with the body surface such as the wrist or the forearm Ar of the patient when the patient moves to twist the wrist or the like.

Furthermore, the lower surface region 132 has the constricted portion 132a in the hemostatic device 10. The constricted portion 132a is formed by decreasing the width of the lower surface region 132 from the first band body 410 side to the curved region 133 side. In the state where the hemostatic device 10 is worn on the right hand H1, the deformation of the upper surface region 131 of the cover member 100 is not inhibited by the lower surface region 132 due to the constricted portion 132a located in the lower surface region 132, and the upper surface region 131 can be deformed along the support member 300. Therefore, in the state where the hemostatic device 10 is worn on the right hand H1, the proximal portion 122 of the cover member 100 is deformed along both ends of the support member 300 in the width direction in the hemostatic device 10, so that a width of the proximal portion 122 of the cover member 100 can be reduced. Since the width of the proximal portion 122 of the cover member 100 decreases, an article or the like is hardly caught on the proximal portion 122 of the cover member 100 in the hemostatic device 10.

Note that, in a case where the pressing member 200 is formed of the inflatable member 210, it is possible to suitably prevent the edge portion 211 of the inflatable member 210 located near the proximal portion 122 of the cover member 100 from coming into contact with a surrounding article such as a table in a state where the inflatable member 210 inflates. However, there is a possibility that the pressing member 200 comes into contact with the surrounding article when the pressing member 200 has a certain thickness or more in a case where the pressing member 200 has a structure other than the inflatable member 210. Therefore, the hemostatic device 10 includes the constricted portion 132a, and thus, the above effect can be suitably exhibited even in the case where the pressing member 200 is configured with the structure other than the inflatable member 210.

Furthermore, in the hemostatic device 10, the thickness on the lower end 302 side of the support member 300 is thinner than the thickness on the upper end 301 side (see Fig. 17). Therefore, when an article such as a table comes into contact from the lower end 302 side of the support member 300 in the state where the hemostatic device 10 is worn on the right hand H1, an impact at the time of the contact can be released from the lower end 302 side to the upper end 301 side. As a result, it is possible to effectively prevent the hemostatic device 10 from being displaced as the article or the like is caught on the lower end 302 of the hemostatic device 10.

After the inflatable member 210 inflates, the operator removes the sheath tube 610 of the introducer 600 from the first puncture site p1 as illustrated in Fig. 16. The operator confirms that there is no bleeding from the first puncture site p1 during hemostasis performed using the hemostatic device 10. In a case where there is bleeding from the first puncture site p1, the operator can adjust an injection amount of air into the inflatable member 210.

According to the above procedure, the operator can stop bleeding at the first puncture site p1 formed on the right hand H1 using the hemostatic device 10.

As described above, the hemostatic device 10 according to the present embodiment includes the cover member 100 configured to cover the first puncture site p1, the inflatable member 210 (pressing member 200) disposed on the cover member 100 and configured to compress the first puncture site p1, and the support member 300 disposed on the cover member 100 so as to cover the inflatable member 210. The cover member 100 includes the main body 110 in which the inflatable member 210 is located, the first band body 410 that is configured to be disposed between fingers of the patient and extends from the main body 110 in the first direction, the second band body 420 extending from the main body 110 in the second direction different from the first direction, and the third band body 430 facing the second band body 420 across the inflatable member 210 and extending from the main body 110 in the third direction different from the first direction and the second direction. The main body 110 includes the retaining portion 130 having a two-layer structure and retaining the support member 300, and the retaining portion 130 includes the upper surface region 131 located on the upper surface 300b side of the support member 300, the lower surface region 132 that is located on a lower surface 300a side of the support member 300 and faces the upper surface region 131 across the support member 300, and a curved region 133 connecting the upper surface region 131 and the lower surface region 132 on the proximal side of the support member 300. The curved region 133 is formed by folding a part of the main body 110 to the first band body 410 side, the lower surface region 132 has the constricted portion 132a on the curved region 133 side, and the width w3 of the lower surface region 132 in the constricted portion 132a decreases from the first band body 410 side to the curved region 133 side.

According to the hemostatic device 10 configured as described above, the main body 110 included in the cover member 100 includes the retaining portion 130 having the two-layer structure to retain the support member 300. The retaining portion 130 has the curved region 133 formed by folding a part of the main body 110 to the first band body 410 side. In the hemostatic device 10, the curved region 133 of the retaining portion 130 comes into contact with the body surface when the patient moves the right hand H1 in the state where the hemostatic device 10 is worn on the right hand H1. Since the curved region 133 formed in a curved shape comes into contact with the body surface of the right hand H1, the hemostatic device 10 can prevent the proximal portion 122 of the cover member 100 from biting into the body surface. Furthermore, the hemostatic device 10 has the lower surface region 132 in which the retaining portion 130 is disposed on the body surface side of the right hand H1. The lower surface region 132 has the constricted portion 132a in which the width of the lower surface region 132 decreases toward the curved region 133 side. Therefore, in the state where the hemostatic device 10 is worn on the right hand H1, the deformation of the upper surface region 131 of the cover member 100 is not inhibited by the lower surface region 132 due to the constricted portion 132a located in the lower surface region 132, and the upper surface region 131 can be deformed along the support member 300. In the state where the hemostatic device 10 is worn on the right hand H1, the proximal portion 122 of the cover member 100 is deformed along both ends of the support member 300 in the width direction in the hemostatic device 10, so that the width of the proximal portion 122 of the cover member 100 decreases. Since the width of the proximal portion 122 of the cover member 100 decreases, the hemostatic device 10 can prevent an article or the like from being easily caught on the proximal portion 122 of the cover member 100.

The support member 300 has the upper end 301 that is located on the first band body 410 side and forms the end of the support member 300 and the lower end 302 located opposite to the upper end 301. The width w2 of the lower end 302 is smaller than the width w1 of the upper end 301. The lower surface 300a of the support member 300 is curved in the shape projecting in the direction away from the inflatable member 210.

According to the hemostatic device 10 configured as described above, the width of the support member 300 decreases from the upper end 301 disposed on the fingertip side to the lower end 302 disposed on the forearm Ar side, and thus, the operator can dispose the support member 300 along the metacarpal bone B1 of the index finger and the metacarpal bone B2 of the thumb by disposing the support member 300 so as to overlap the metacarpal bone B1 of the index finger and the metacarpal bone B2 of the thumb from the dorsal side of the right hand H1. The operator can prevent a gap from being formed between the inflatable member 210 disposed on the lower surface 300a side of the support member 300 and the right hand H1 by disposing the support member 300 along the metacarpal bone B1 of the index finger and the metacarpal bone B2 of the thumb.

Furthermore, according to the hemostatic device 10 configured as described above, the width w2 of the lower end 302 of the support member 300 is smaller than the width w1 of the upper end 301 of the support member 300, and thus, the support member 300 can be disposed along the metacarpal bone B1 of the index finger and the metacarpal bone B2 of the thumb when the support member 300 is disposed on the right hand H1. Therefore, the hemostatic device 10 can firmly wrap the second band body 420 and the third band body 430 along the outer periphery of the right hand H1. Further, in the hemostatic device 10, the lower surface 300a of the support member 300 is curved in the shape projecting in the direction away from the inflatable member 210. Therefore, the inflatable member 210 is curved so as to fit along the lower surface 300a of the support member 300 in the state where the hemostatic device 10 is worn on the right hand H1. Therefore, it is possible to effectively increase the compressive force applied to the first puncture site p1 by the inflatable member 210.

The support member 300 has the side surface portions 303 and 304 connecting the upper end 301 and the lower end 302. The thickness t1 of each of the side surface portions 303 and 304 decreases from the upper end 301 to the lower end 302.

According to the hemostatic device 10 configured as described above, when the inflatable member 210 starts to inflate in a state where the support member 300 and the inflatable member 210 are secured to the right hand H1, the inflatable member 210 applies a force to the vicinity of the upper end 301 of the support member 300. The upper end 301 side of the support member 300 is lifted by the force applied by the inflatable member 210. As a result, the support member 300 is disposed such that the lower surface 300a of the support member 300 is substantially parallel with the body surface of the right hand H1 in a state where the inflatable member 210 inflates. Therefore, the hemostatic device 10 can apply a compressive force in a direction perpendicular to the first puncture site p1 when the inflatable member 210 inflates. Furthermore, since the thickness on the lower end 302 side of the support member 300 is thinner than the thickness on the upper end 301 side in the hemostatic device 10, when an article such as a table comes into contact from the lower end 302 side of the support member 300 in the state where the hemostatic device 10 is worn on the right hand H1, the impact at the time of the contact can be released from the lower end 302 side to the upper end 301 side. As a result, it is possible to effectively prevent the hemostatic device 10 from being displaced as the article or the like is caught on the lower end 302 of the hemostatic device 10.

Furthermore, the width w3 of the curved region 133 is smaller than the width w2 of the proximal portion of the support member 300 in the plan view.

As described above, the width w3 of the curved region 133 is smaller than the width w2 of a proximal end of the support member 300 in the plan view of the hemostatic device 10. Therefore, in the hemostatic device 10, the upper surface region 131 is deformed along both the ends of the support member 300 in the width direction by the constricted portion 132a located in the lower surface region 132 at the proximal portion 122 of the cover member 100 in a state where the hemostatic device 10 is worn on the right hand H1 and the inflatable member 210 inflates. As a result, the hemostatic device 10 can further reduce the width of the proximal portion 122 of the cover member 100. As a result, the hemostatic device 10 can more reliably prevent an article or the like from being easily caught on the proximal portion 122 of the cover member 100.

The retaining portion 130 is located on the proximal side of the main body 110. The inflatable member 210 is connected to the lower surface region 132 such that a part of the inflatable member 210 is located at the constricted portion 132a.

According to the hemostatic device 10 configured as described above, the center c1 of the inflatable member 210 is located at a position shifted to the proximal portion side of the main body 110 with respect to the center of the main body 110 in the longitudinal direction. Furthermore, the center c1 of the inflatable member 210 is located at a position shifted to the proximal portion side of the main body 110 from the center of gravity G1 of the support member 300. Therefore, it is possible to prevent the inflatable member 210 from lifting-off on the distal portion side of the main body 110 when the second band body 420 and the third band body 430 are wrapped along the outer periphery of the right hand H1 in order to cause the hemostatic device 10 to be worn on the right hand H1. As a result, the hemostatic device 10 can prevent the compressive force applied to the first puncture site p1 by the inflatable member 210 from decreasing as the inflatable member 210 lifts-off on the distal portion side of the main body 110.

In the inflatable member 210, the edge portion 211 located on the proximal side of the inflatable member 210 is located in the curved region 133.

According to the hemostatic device 10 configured as described above, the center c1 of the inflatable member 210 is located at a position shifted to the curved region 133 side located at the proximal portion 122 of the cover member 100. As a result, since the center c1 of the inflatable member 210 is disposed on the proximal portion 122 side of the cover member 100 with respect to the center of gravity G1 of the support member 300 in the hemostatic device 10, it is possible to more reliably prevent the inflatable member 210 from lifting-off on the distal portion side of the main body 110 when the second band body 420 and the third band body 430 are wrapped along the outer periphery of the right hand H1.

The curved region 133 has the curved portions 133a located at both the ends in the width direction of the curved region 133.

According to the hemostatic device 10 configured as described above, when a load is applied to both the ends in the width direction of the curved region 133 by the patient moving to twist the wrist or the like, the curved portion 133a alleviates the concentration of the load on the constricted portion 132a. Therefore, it is possible to prevent the curved region 133 from being damaged near the constricted portion 132a when both the ends in the width direction of the curved region 133 are deformed to be bent.

### <Modification>

Next, a modification of the first embodiment will be described. In the description of the modifications, the description regarding the members and the process of using the hemostatic device already described in the first embodiment will be appropriately omitted. Furthermore, contents that are not particularly described in the modifications can be the same as those in the first embodiment.

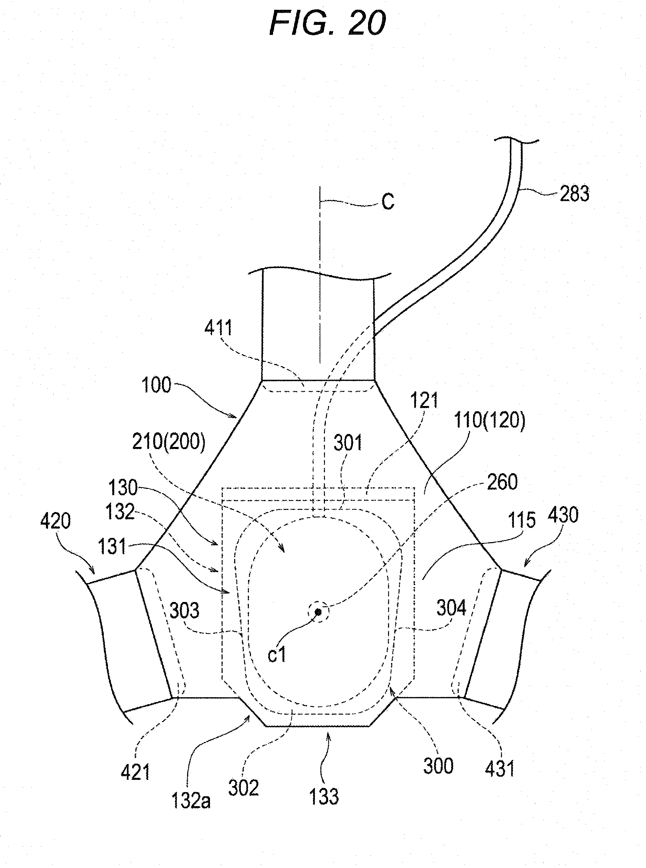

Fig. 20 illustrates a part of a hemostatic device according to the modification in an enlarged manner. Fig. 20 is a plan view corresponding to Fig. 5 described in the hemostatic device 10 according to the first embodiment.

In the hemostatic device 10 according to the modification, the curved region 133 is disposed at a position protruding to the proximal side from the proximal portion of the cover member 100. Specifically, the curved region 133 is disposed at a position protruding to the proximal side from the intermediate region 115 of the main body 110 located between the second band body 420 and the third band body 430.

A part of the support member 300 and a part of the inflatable member 210 are disposed on the proximal side of the hemostatic device 10 according to the first embodiment in accordance with the curved region 133 being disposed on the proximal side of the intermediate region 115 of the main body 110. Specifically, the lower end 302 of the support member 300 and the edge portion 211 on the proximal side of the inflatable member 210 are disposed on the proximal side of the intermediate region 115 of the main body 110.

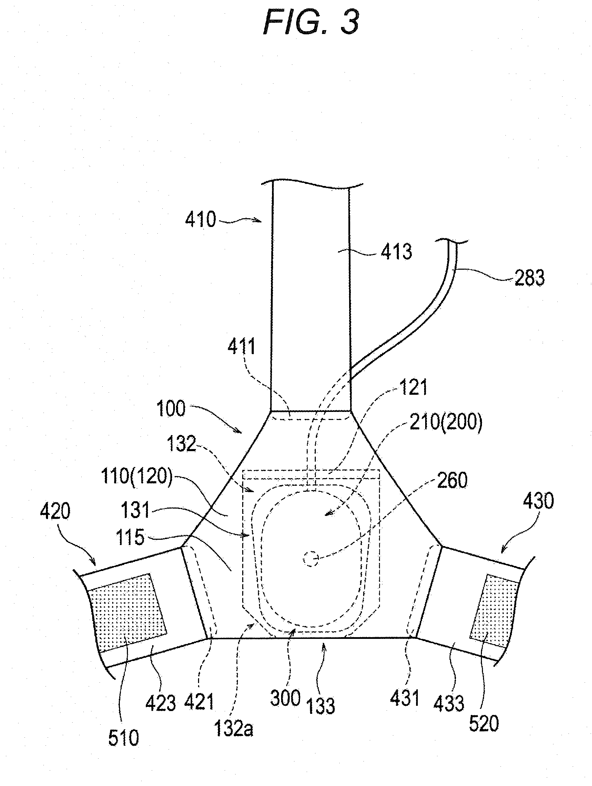

The curved region 133 can be disposed on the proximal side of the intermediate region 115 of the main body 110 as in the present modification. When the curved region 133 is disposed in this manner, the inflatable member 210 connected to the lower surface region 132 of the retaining portion 130 can be disposed at a position closer to the proximal side of the cover member 100. Therefore, the hemostatic device according to the modification can more reliably prevent the inflatable member 210 from lifting-off on the distal portion side of the main body 110 when the second band body 420 and the third band body 430 are wrapped along the outer periphery of the right hand H1. However, in the present modification, a part of the support member 300 formed using a hard material protrudes so as to be located on the proximal side of the intermediate region 115 of the main body 110 as illustrated in Fig. 20. Therefore, a position on the proximal side of the cover member 100 is more easily caught by an article or the like in the present modification as compared with the first embodiment in Figs. 1 and 3.

From the above viewpoint, there is a case where it is more preferable to adopt the structure of the first embodiment in the hemostatic device.

Note that, in a case where a position of the curved region 133 and a position of the inflatable member 210 are changed as in the hemostatic device according to the present modification, a longitudinal length of the retaining portion 130 increases as the position of the curved region 133 is shifted to the proximal side. Therefore, in the hemostatic device according to the present modification, it is possible to use the support member 300 having the same longitudinal length as that in the hemostatic device 10 according to the first embodiment in order to dispose the support member 300 so as to overlap the entire inflatable member 210 in a plan view.

In the hemostatic device according to the modification, a position of the first end 121 can also be disposed at the same position as that in the above embodiment. That is, when the curved region 133 is disposed on the proximal side of the intermediate region 115 of the main body 110 without shifting the position of the first end 121 to the proximal side of the main body 110, the retaining portion can be formed to have a large longitudinal length. However, with such a configuration, the support member disposed in the retaining portion needs to be formed to have a large longitudinal length in accordance with the longitudinal length of the retaining portion. When the support member is formed to have the large longitudinal length, it becomes difficult to appropriately align an arbitrary position of the support member in the longitudinal direction with the first puncture site p1. Therefore, as described in the above modification, the position of the first end 121 is preferably disposed at the position shifted to the proximal side of the main body 110 in accordance with the curved region 133 being disposed on the proximal side of the intermediate region 115 of the main body 110.

### <Second Embodiment>

Next, a hemostatic device according to a second embodiment will be described. In the description of the second embodiment, the description regarding the members and the process of using the hemostatic device already described in the first embodiment will be appropriately omitted. Furthermore, contents that are not particularly described in the modifications can be the same as those in the first embodiment.

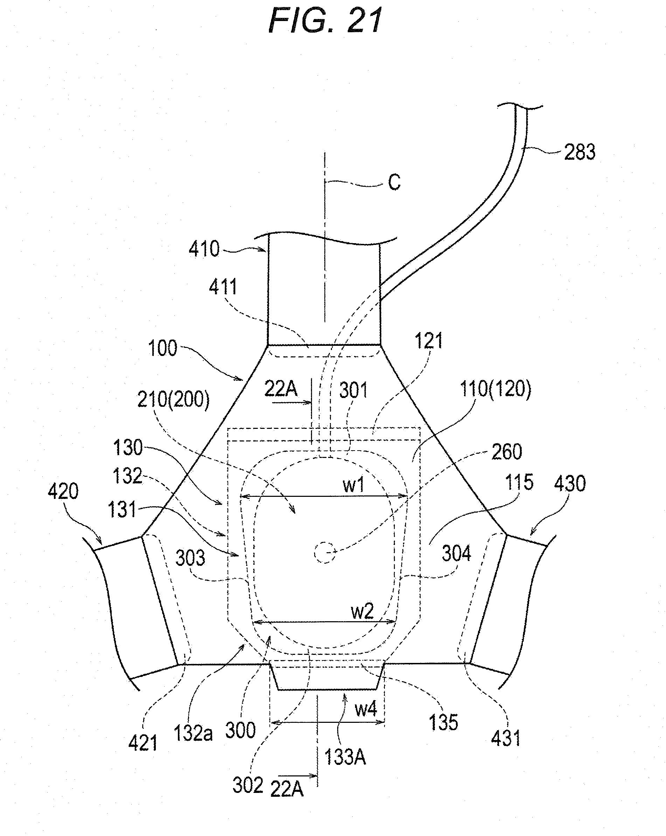

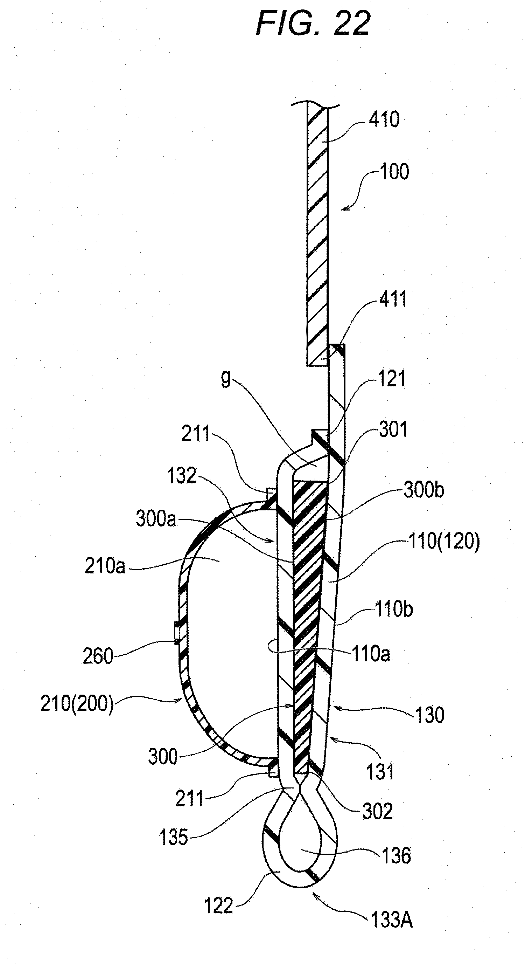

Fig. 21 illustrates a part of the hemostatic device according to the second embodiment in an enlarged manner. Fig. 21 is a plan view corresponding to Fig. 5 described in the hemostatic device 10 according to the first embodiment. Fig. 22 is a cross-sectional view taken along an arrow 22A-22A illustrated in Fig. 21.

The hemostatic device according to the second embodiment is different from the hemostatic device 10 according to the first embodiment in terms of a structure of a curved region 133A. Hereinafter, the hemostatic device according to the second embodiment will be described.

As illustrated in Fig. 21, the hemostatic device according to the second embodiment includes the cover member 100 configured to cover the first puncture site p1, the inflatable member 210 connected to the cover member 100 and configured to compress the first puncture site p1, and the support member 300 disposed on the cover member 100 so as to cover the inflatable member 210.

As illustrated in Fig. 21, the cover member 100 includes the main body 110 in which the inflatable member 210 is located, the first band body 410 that is configured to be disposed between fingers of a patient and extends from the main body 110 in a first direction, the second band body 420 extending from the main body 110 in a second direction different from the first direction, and the third band body 430 facing the second band body 420 across the inflatable member 210 and extending from the main body 110 in a third direction different from the first direction and the second direction.

As illustrated in Fig. 22, the main body 110 includes the retaining portion 130 that has a two-layer structure and retains the support member 300.

As illustrated in Fig. 22, the retaining portion 130 includes the upper surface region 131 located on the upper surface 300b side of the support member 300, the lower surface region 132 that is located on the lower surface 300a side of the support member 300 and faces the upper surface region 131 across the support member 300, a connection region 135 connecting the upper surface region 131 and the lower surface region 132 on a proximal side of the lower end (proximal portion) 302 of the support member 300, and the curved region 133A connecting the upper surface region 131 and the lower surface region 132 on the proximal side of the connection region 135 and forming a space portion 136 between the upper surface region 131 and the lower surface region 132.

As illustrated in Fig. 22, the curved region 133A is formed by folding a part of the sheet material 120 forming the main body 110 toward the first band body 410 side (distal side).

As illustrated in Fig. 21, the constricted portion 132a can be provided in the lower surface region 132 of the retaining portion 130.

A width w4 of the connection region 135 is smaller than the width w2 of the lower end 302 of the support member 300 in the plan view illustrated in Fig. 21. Therefore, the upper surface region 131 is deformed along both ends of the support member 300 in the width direction by the constricted portion 132a located in the lower surface region 132 on a proximal portion side of the cover member 100 in a state where the hemostatic device is worn on the right hand H1 where the first puncture site p1 is formed and the inflatable member 210 inflates. Therefore, in the hemostatic device, a width on the proximal portion side of the cover member 100 can be further reduced. As a result, the hemostatic device can more reliably prevent an article or the like from being easily caught on the proximal portion side of the cover member 100.

As illustrated in Figs. 21 and 22, the hollow space portion 136 is formed inside the curved region 133A. The curved region 133A comes into contact with a body surface of a wrist or the forearm Ar of the patient when the patient moves to twist the wrist toward a dorsal side of the right hand H1 or the like in a state where the hemostatic device 10 is worn on the right hand H1. Since the space portion 136 is formed inside the curved region 133A in the hemostatic device according to the second embodiment, the curved region 133 is flexibly deformed when the curved region 133A comes into contact with the body surface of the wrist or the forearm Ar of the patient. Therefore, the hemostatic device according to the second embodiment can prevent the curved region 133 from biting into the body surface.

Furthermore, the hemostatic device according to the second embodiment can retain an accessory or the like (a member having a clip structure or the like) of the hemostatic device 10 in the cover member 100 by catching the accessory in the space portion 136.

Although the hemostatic device according to the present invention has been described above through the plurality of embodiments and modifications, the present invention is not limited only to the content described in the specification and can be appropriately changed based on the description of the claims.

The shape, size, and the like of each part of the hemostatic device are not particularly limited as long as a puncture site can be compressed to stop bleeding by a pressing member disposed at the puncture site, and can be appropriately changed.

Specific shapes (planar shape and cross-sectional shape) of the inflatable member and the support member are not limited to the shapes illustrated in the embodiments.

A configuration of the pressing member that applies a compressive force to the puncture site is not limited to the inflatable member (balloon). The pressing member can also be configured using, for example, a resin material such as plastic configured to be capable of applying the compressive force to the puncture site, a member made of gel or the like, an elastic material such as a sponge-like substance, an assembly of fibers such as cotton, metal, a member having a predetermined three-dimensional shape (a sphere, an ellipsoid, a triangular pyramid, or the like), or one obtained by combining these as appropriate.

The hemostatic device can also be configured for the purpose of hemostasis other than the puncture site formed on the hand. For example, the hemostatic device can also be applied to hemostasis of a puncture site formed on an arm, a leg, or the like of a patient using the inflatable portion of the embodiments.

The present application is based on Japanese Patent Application No. 2022-44652 filed on March 18, 2022, the disclosure content of which is incorporated herein by reference in its entirety.

### Reference Signs List

10 Hemostatic device
100 Cover member
110 Main body
110a Inner surface of main body
110b Outer surface of main body
115 Intermediate region
120 Sheet material
122 Proximal portion of cover member
130 Retaining portion
131 Upper surface region
132 Lower surface region
132a Constricted portion
133 Curved region
133A Curved region
133a Curved portion
135 Connection region
136 Space portion
200 Pressing member
210 Inflatable member
210a Inner cavity
211 Edge portion
260 Marker portion
300 Support member
300a Lower surface of support member
300b Upper surface of support member
301 Upper end
302 Lower end
303 Side surface portion
304 Side surface portion
305 Intermediate portion
410 First band body
420 Second band body
430 Third band body
600 Introducer
g Insertion portion
C Center line of first band body
c1 Center of inflatable member
wa Longitudinal width of inflatable member
wb Lateral width of inflatable member
t1 Thickness of side surface portion
w1 Width of upper end
w2 Width of lower end
w3 Width of curved region
G1 Center of gravity of support member
H Hand
H1 Right hand
Ar Forearm
fb Interdigital part
Sb Snuff box
B1 Metacarpal bone of index finger
B2 Metacarpal bone of thumb
T1 Extensor pollicis longus muscle
T2 Extensor pollicis brevis muscle
V1 Blood vessel (distal radial artery)
V2 Artery located at snuff box
p1 First puncture site (puncture site)
p2 Second puncture site (puncture site)

## Claims

1. A hemostatic device comprising:
a cover member configured to cover a puncture site formed on a patient;
a pressing member disposed on the cover member and configured to compress the puncture site; and
a support member disposed on the cover member to cover the pressing member, wherein
the cover member includes a main body in which the pressing member is located, a first band body which is configured to be disposed between fingers of the patient and extends in a first direction from the main body, a second band body extending in a second direction different from the first direction from the main body, and a third band body facing the second band body across the pressing member and extending in a third direction different from the first direction and the second direction from the main body,
the main body includes a retaining portion that has a two-layer structure and retains the support member,
the retaining portion includes an upper surface region located on an upper surface side of the support member, a lower surface region that is located on a lower surface side of the support member and faces the upper surface region across the support member, and a curved region connecting the upper surface region and the lower surface region on a proximal side of the support member,
the curved region is formed by folding a part of the main body toward a side where the first band body is disposed,
the lower surface region includes a constricted portion on a side where the curved region is disposed, and
a width of the lower surface region in the constricted portion decreases from the side where the first band body is disposed to the side where curved region is disposed.

2. The hemostatic device according to claim 1, wherein
the support member includes an upper end, which is located on the side where the first band body is disposed and forms an end of the support member, and a lower end forming an end opposite to the upper end,
a width of the lower end is smaller than a width of the upper end, and
the lower surface of the support member is curved in a shape projecting in a direction away from the pressing member.

3. The hemostatic device according to claim 2, wherein
the support member has a side surface portion connecting the upper end and the lower end, and
a thickness of the side surface portion decreases from the upper end to the lower end.

4. The hemostatic device according to claim 2 or 3, wherein a width of the curved region is smaller than a width of a proximal portion of the support member in a plan view.

5. The hemostatic device according to any one of claims 1 to 4, wherein
the retaining portion is located on a proximal side of the main body, and
the pressing member is connected to the lower surface region in such a manner that a part of the pressing member is located in the constricted portion.

6. The hemostatic device according to any one of claims 1 to 5, wherein the pressing member has an edge portion located on a proximal side of the pressing member, the edge portion being located in the curved region.

7. The hemostatic device according to any one of claims 1 to 6, wherein the curved region has curved portions located at both ends in a width direction of the curved region.

8. A hemostatic device comprising:
a cover member configured to cover a puncture site formed on a patient;
a pressing member connected to the cover member and configured to compress the puncture site; and
a support member disposed on the cover member to cover the pressing member, wherein
the cover member includes a main body in which the pressing member is located, a first band body which is configured to be disposed between fingers of the patient and extends in a first direction from the main body, a second band body extending in a second direction different from the first direction from the main body, and a third band body facing the second band body across the pressing member and extending in a third direction different from the first direction and the second direction from the main body,
the main body includes a retaining portion that has a two-layer structure and retains the support member,
the retaining portion includes an upper surface region located on an upper surface side of the support member, a lower surface region that is located on a lower surface side of the support member and faces the upper surface region across the support member, a connection region connecting the upper surface region and the lower surface region on a proximal side of a proximal portion of the support member, and a curved region connecting the upper surface region and the lower surface region on the proximal side of the connection region and forming a space portion between the upper surface region and the lower surface region, and
the curved region is formed by folding a part of the main body toward a side where the first band body is disposed.
